# EUROPÄISCHE PATENTSCHRIFT

(11) **EP 1 335 685 B1**
(45) Veröffentlichungstag und Bekanntmachung des Hinweises auf die Patenterteilung: **12.12.2007**
(21) Anmeldenummer: 01997267.8
(22) Anmeldetag: 17.10.2001
(51) Int. Cl.: A61F 2/38

(54) **GELENKPROTHESE**
JOINT PROSTHESIS
PROTHESE ARTICULAIRE

(30) Priorität: 24.11.2000 DE 10058372
(43) Veröffentlichungstag der Anmeldung: 20.08.2003
(73) Patentinhaber: Mathys Medizinaltechnik AG, 2544 Bettlach (CH)
(72) Erfinder: DELFOSSE, Daniel, CH- Bern 3018 (CH); SUPPER, Walter, CH-2544 Bettlach (CH); GRUNDER, Beat, CH-3076 Worb (CH)
(74) Vertreter: Körfer, Thomas
(86) Internationale Anmeldenummer: PCT/EP2001/012026
(87) Internationale Veröffentlichungsnummer: WO 2002/041810

(56) Entgegenhaltungen:
- WO-A-92/08424
- US-A- 6 068 658
- US-A- 6 123 728

## Beschreibung

Die Erfindung betrifft eine Gelenkprothese zum Ausbilden einer Gelenkverbindung zwischen einem Paar menschlicher oder tierischer Knochen.

Aus der EP 0 510 178 B1 ist eine Gelenkprothese mit den Merkmalen des Oberbegriffs des Anspruchs 1 bekannt. Die bekannte Gelenkprothese ist als Kniegelenkprothese ausgebildet zum Ausbilden einer Gelenkverbindung zwischen einem ersten Knochen (Tibia) und einem zweiten Knochen (Femur). Dabei sind eine erste Komponente, die mit dem ersten Knochen verbunden ist, und eine zweite Komponente vorgesehen, auf der sich der zweite Knochen mittels eines Abstützmittels abstützt und die auf einer Lagerfläche der ersten Komponente bewegbar gelagert ist. Ferner ist ein mit der ersten Komponente verbundenes Führungselement, das in eine Durchgangsöffnung der zweiten Komponente eingreift, vorgesehen, um eine translative Bewegung der zweiten Komponente relativ zu der ersten Komponente zu ermöglichen, wobei die Translationsbewegung durch an der Durchgangsöffnung ausgebildete Innenflächen in anterior-posterior Richtung begrenzt.

Die aus der EP 0 510 178 B1 bekannte Gelenkprothese hat den Nachteil, daß das Führungselement an Seitenflächen der Durchgangsöffnung anschlägt, die Teil der zweiten Komponente sind. Da die zweite Komponente außerdem ein gleitendes Abstützen des Abstützmittels, mit dem der zweite Knochen an der zweiten Komponente abgestützt ist, gewährleisten muß, sind hohe Anforderungen an das Material der zweiten Komponente zu stellen, wobei insbesondere ein geeigneter Kompromiß zwischen Festigkeit und vorteilhaften Gleiteigenschaften eingegangen werden muß. Beispielsweise wird die zweite Komponente, um die Gleiteigenschaften sowohl bezüglich der Lagerfläche der ersten Komponente als auch bezüglich des Abstützmittels zu gewährleisten, aus einem geeigneten Kunststoff gefertigt, der nur eine begrenzte Festigkeit aufweist, so daß insbesondere die als Anschlag dienenden Seitenflächen der Durchgangsöffnung nach einer hohen Wiederholungszahl der anterior-posterior Bewegung übermäßig beansprucht werden.

Ein weiterer Nachteil ist, daß die Länge der durch die Größe der Durchgangsöffnung vorgegebenen Führungsbahn, d.h. der Bewegungsbereich der anterior-posterior Bewegung der Gelenkprothese, durch die bauliche Ausgestaltung der zweiten Komponente fest vorgegeben ist. Daher sind für andere Anforderungen, bei denen eine Gelenkprothese mit kürzerer oder längerer Bewegungsfreiheit in anterior-posterior Richtung oder eine Gelenkprothese, die nur eine Drehbewegung erlaubt, erforderlich sind, andere Gelenkprothesen zu verwenden, wofür insbesondere eine vollständige Neukonstruktion der Gelenkprothese erforderlich sein kann.

Der Erfindung liegt die Aufgabe zugrunde, eine Gelenkprothese bereitzustellen, bei der eine translative Bewegung, insbesondere eine Bewegung in anterior-posterior Richtung, unabhängig von der Bauform der zweiten Komponente in einfacher Weise vorgebbar ist, und bei der insbesondere die Belastung der zweiten Komponente verringert ist.

Die Aufgabe wird durch eine Gelenkprothese mit den Merkmalen des Anspruchs 1 gelöst. Vorteilhafte Weiterbildungen der Erfindung sind durch die in den Unteransprüchen angegebenen Maßnahmen möglich.

Die erfindungsgemäße Gelenkprothese hat den Vorteil, daß das Führungselement mittels eines in der zweiten Komponente drehbar gelagerten Lagerkörpers in die zweite Komponente eingreift. Dadurch kann das Material des Führungselements unabhängig von dem Material der zweiten Komponente gewählt werden. Zum Beispiel kann das Material des Lagerkörpers so gewählt werden, daß eine leichtgängige und verschleißarme Führung zwischen dem Führungselement und dem Lagerkörper besteht, während das Material der zweiten Komponente in Bezug auf eine vorteilhafte Abstützung des zweiten Knochens an der zweiten Komponente gewählt ist. Dabei ermöglicht der Lagerkörper eine Drehung des Führungselements relativ zu der zweiten Komponente, wodurch sich eine vorteilhafte Aufteilung zwischen der translativen Beweglichkeit und der Drehbeweglichkeit ergibt, bei der die Drehung stets um die durch den Lagerkörper vorgegebene Achse erfolgt.

Vorteilhaft ist es, daß der Lagerkörper eine zylinderförmige Grundform aufweist. Dadurch ist eine vorteilhafte Kraftübertragung zwischen dem Lagerkörper und der ersten Komponente gegeben.

In vorteilhafter Weise ist der Lagerkörper in einer zumindest im wesentlichen zylinderförmig ausgebildeten Aussparung der zweiten Komponente gelagert. Dadurch ist ein Lager mit einfacher Geometrie gegeben, das eine maximale Drehbeweglichkeit ermöglicht und in jeder Drehstellung eine vorteilhafte. Kraftübertragung und geringen Verschleiß gewährleistet.

Vorteilhaft ist es, daß der Lagerkörper zumindest einen seitlichen Bund aufweist, der in eine mit der zylinderförmigen Aussparung verbundenen Nut der zweiten Komponente eingreift. Dadurch ist eine einfache, gegebenenfalls auch unlösbare, Verbindung des Lagerkörpers in der zweiten Komponente gegeben, die keinen negativen Einfluß auf die Drehbeweglichkeit des Lagerkörpers hat.

Vorteilhaft ist es, daß der seitliche Bund des Lagerkörpers als umlaufender Kreisbund ausgebildet ist. Dadurch ist eine vorteilhafte Anlagefläche des seitlichen Bundes des Lagerkörpers an der mit der zylinderförmigen Aussparung verbundenen Nut der zweiten Komponente, die sich radial vollständig umlaufend erstreckt, gegeben, so daß sich eine gleichmäßige Belastung und eine maximale Haltekraft ergibt.

In vorteilhafter Weise ist der seitliche Bund des Lagerkörpers als abschnittsweise umlaufender Kreisbund ausgebildet. Dabei ist es besonders vorteilhaft, daß die in der zweiten Komponente ausgebildete Nut umfänglich abschnittsweise ausgebildet ist, um die Drehbewegung des Führungselementes relativ zu der zweiten Komponente zu begrenzen. Dadurch kann ein Drehwinkelbereich vorgegeben werden, innerhalb dem eine Drehbewegung der ersten Komponente relativ zu der zweiten Komponente möglich ist, um eine Anpassung an den anatomischen Bewegungsablauf des durch die Gelenkprothese ersetzten Gelenks zu erzielen.

Vorteilhaft ist es, daß die in der zweiten Komponente ausgebildete Nut umfänglich umlaufend ausgebildet ist, um eine unbegrenzte Drehbeweglichkeit des Führungselementes relativ zu der zweiten Komponente zu gewährleisten. Dies ist unabhängig davon, ob der seitliche Bund des Lagerkörpers abschnittsweise oder vollständig umlaufend ausgebildet ist, die Zusammenwirkung mit dem vollständig umlaufenden Kreisbund des Lagerkörpers ist allerdings besonders vorteilhaft.

Vorteilhaft ist es, daß der Lagerkörper zumindest eine erste Aussparung aufweist, in der das Führungselement geführt ist. Die Aussparung kann dabei im wesentlichen innerhalb des Lagerkörpers oder aber auch als eine auf der Seite der Lagerfläche der ersten Komponente angeordnete erste Nut ausgebildet sein. Für den Fall, daß die Aussparung zumindest abschnittsweise zur Seite der Lagerfläche der ersten Komponente geschlossen ist, wird zumindest für bestimmte Stellungen des Lagerkörpers in der Aussparung die zweite Komponente an der Lagerfläche der ersten Komponente gehalten.

Vorteilhaft ist es, daß das Führungselement in der ersten Aussparung in einer Längsrichtung der ersten Aussparung bewegbar ist, um eine translative Bewegung des Führungselementes zu ermöglichen. Dabei ist die Aussparung in der Längsrichtung länger als das Führungselement ausgebildet. Dadurch weist die Gelenkprothese eine Beweglichkeit der zweiten Komponente relativ zu der ersten Komponente in der Längsrichtung, insbesondere in anterior-posterior Richtung, auf.

Ferner ist es vorteilhaft, daß die erste Aussparung zumindest in einer Längsrichtung eine Anschlagfläche aufweist, die die translative Bewegung des Führungselementes in der ersten Aussparung begrenzt. Dadurch kann der Grad der Einschränkung der translativen Bewegungsmöglichkeit der Gelenkprothese vorgegeben werden. Außerdem wird dadurch erreicht, daß das Führungselement an einer Anschlagfläche des Lagerkörpers anschlägt, so daß die Belastung der zweiten Komponente verringert ist. Die translative Bewegung kann auch durch zwei Anschlagflächen in beiden Richtungen begrenzt werden, was besonders vorteilhaft ist.

Vorteilhaft ist es außerdem, daß der Lagerkörper zumindest eine seitlich der Anschlagfläche der ersten Aussparung angeordnete Kantenausformung aufweist, die mit der ersten Aussparung verbunden ist. Dadurch verteilt sich beim Anschlagen des Führungselements die Anschlagkraft gleichmäßig über die Anschlagfläche, wobei eine Belastung im Kantenbereich der Aussparung verhindert ist. Die Kantenausformung kann z.B. als Bohrung ausgebildet sein.

Vorteilhaft ist es, daß der Lagerkörper zumindest eine weitere zweite Aussparung aufweist, in die das Führungselement einsetzbar ist, wobei der Lagerkörper in der zweiten Aussparung zumindest im wesentlichen fixiert ist. Die zweite Aussparung kann z.B. senkrecht zur ersten Aussparung angeordnet werden, so daß beim Einbau der Gelenkprothese entschieden werden kann, ob eine translative Bewegung des Führungselementes und damit eine Translationsbewegung der Gelenkprothese bezüglich Verschiebung der zweiten Komponente relativ zu der ersten Komponente möglich sein soll oder nicht. Alternativ dazu kann der Lagerkörper auch nur eine solche zweite Aussparung aufweisen, wobei bei einer solchen Gelenkprothese nur eine Drehbeweglichkeit gegeben ist. Die zweite Aussparung kann entsprechend den vorteilhaften Weiterbildungen der ersten Aussparung weitergebildet werden.

In vorteilhafter Weise, weist das Führungselement einen im wesentlichen rechteckigen Querschnitt auf. Dadurch sind längsseits an dem Führungselement vorteilhafte Führungsflächen ausgebildet, die eine großflächige Führung erlauben, während stirnseitig vorteilhafte Anschlagflächen ausgebildet sind, die eine gleichmäßige Kraftübertragung der Anschlagkraft beim Anschlagen des Führungselements an der zweiten Komponente oder der Anschlagfläche des Lagerkörpers ermöglichen.

Ferner ist es vorteilhaft, daß das Führungselement einen Querschnitt mit abgerundeten Kanten aufweist. Dadurch wird die Abnutzung der Führungsflächen des Lagerkörpers, an denen das Führungselement geführt ist, verringert und beim Anschlagen des Führungselements an einer Anschlagfläche, insbesondere an einer Seitenwand der zylinderförmig ausgebildeten Aussparung der zweiten Komponente, sichergestellt, daß eine gleichmäßige Übertragung der Anschlagkraft gewährleistet und eine Beschädigung der Anschlagfläche verhindert ist.

Vorteilhaft ist es ferner, daß das Führungselement einen Querschnitt mit zumindest einem abgerundeten Ende aufweist. Dies ist insbesondere für den Fall vorteilhaft, daß das Führungselement mit dem abgerundeten Ende an eine abgerundete, insbesondere zylindermantelförmige, Anschlagfläche anschlägt, z.B. wenn die Anschlagfläche durch die Innenfläche der zylinderförmig ausgebildeten Aussparung der zweiten Komponente gegeben ist. Für diesen Fall ist es vorteilhaft, daß der Krümmungsradius des abgerundeten Endes des Führungselements zumindest im wesentlichen gleich dem Radius der zylinderförmig ausgebildeten Aussparung ist.

Vorteilhaft ist es ferner, daß der Drehpunkt, um den sich die zweite Komponente bezüglich der ersten Komponente dreht, auf der Seite des Femurs angeordnet ist. Dies ist aus physiologischer Sicht besonders günstig und vermeidet Bänderdehnungen bei der Kniebewegung.

Ein weiterer Vorteil ist, daß der Lagerkörper sowohl die Aussparung 31 als auch die Aussparung 32 aufweist, die vorzugsweise senkrecht zueinander ausgebildet sind. Dadurch kann der Arzt interoperativ entscheiden, welche der beiden Aussparungen, insbesondere mit oder ohne Anschlag, er je nach Zustand der Bänder des Patienten verwenden möchte. Beispielsweise kann der Arzt in Abhängigkeit von dem Zustand der Bänder entscheiden, ob die Gelenkprothese eine Drehbewegung und eine translative Bewegung oder nur eine Drehbewegung ermöglichen soll.

Vorteilhaft ist es, daß das Führungselement mit einem Verriegelungskopf verbunden ist, der in den Lagerkörper eingreift, um die erste Komponente in dem Lagerkörper zu verriegeln. Dadurch ist bei der Gelenkprothese im montierten Zustand die erste Komponente mit der zweiten Komponente verbunden, um ein Lösen der zweiten Komponente zu verhindern. Dabei kann der Verriegelungskopf die zweite Komponente mittelbar mit der ersten Komponente verbinden. Z. b. kann der Verriegelungskopf in das Lagerelement eingreifen, um dieses bezüglich der ersten Komponente zu sichern, während der Lagerkörper mit der zweiten Komponente verbunden ist, so daß die zweite Komponente mittels des Lagerelementes mit der ersten Komponente verbunden ist. Der Verriegelungskopf kann allerdings auch direkt in die zweite Komponente eingreifen, so daß eine unmittelbare Sicherung der zweiten Komponente an der ersten Komponente besteht. Die Verriegelung kann, insbesondere in einer vorgegebenen Drehwinkelstellung des Verriegelungskopfes bezüglich des Lagerkörpers und/oder in einer vorgegebenen Drehwinkelstellung bezüglich der zweiten Komponente, entriegelt sein, um eine Demontage der Gelenkprothese zu ermöglichen. Der Verriegelungskopf kann allerdings auch in den Lagerkörper und oder die erste Komponente einrasten, so daß eine durch den Verriegelungskopf geschaffene unlösbare Verbindung zwischen der zweiten Komponente und der ersten Komponente bzw. zwischen dem Lagerkörper und der ersten Komponente gegeben ist.

In vorteilhafter Weise ist die Längsachse des Verriegelungskopfes bezüglich der Längsachse des Führungselementes um einen Schwenkwinkel verschwenkt. Die Montage der Gelenkprothese kann dann erfolgen, indem die Längsachse des Verriegelungskopfes zunächst mit einer Längsachse des Lagerkörpers überlagert wird und danach das Einfügen des Verriegelungskopfes in den Lagerkörper in einer Position, in der die Achse des Führungselements mit der Achse des Lagerkörpers übereinstimmt, erfolgt. Somit ist eine einfache Verriegelung gegeben, die während der Operation der Gelenkprothese zuverlässig und einfach erreicht werden kann, wobei eine Fehlmontage ausgeschlossen ist. Dabei ist es besonders vorteilhaft, daß die Längsachse des Verriegelungskopfes bezüglich der Längsachse des Führungselementes um einen Schwenkwinkel verschwenkt ist, wobei die Verschwenkung vorzugsweise in einer Ebenen erfolgt, die parallel zur Lagerfläche der ersten Komponente ist.

Vorteilhaft ist es, daß der Lagerkörper zumindest eine Führungsfläche aufweist, an der der Verriegelungskopf bei einer translativen Bewegung der zweiten Komponente relativ zu der ersten Komponente an seiner Unterseite geführt ist. Dadurch ist bei Zugbelastungen, die auf die Gelenkprothese einwirken, eine Führung in der Gelenkprothese gegeben, die Reibungsverluste verringert und ein mögliches Spiel der Gelenkprothese in einer Richtung, die senkrecht zur Lagerfläche der ersten Komponente ist, begrenzt bzw. verhindert.

Vorteilhaft ist es, daß die zweite Komponente zumindest einen Längskörper aufweist, der zumindest eine Längsfläche umfaßt, und daß die Drehung des Führungselementes relativ zu der zweiten Komponente durch Anschlagen zumindest einer Längsseite des Lagerkörpers an der Längsfläche des Längskörpers begrenzt ist. Dadurch kann die Drehbeweglichkeit der zweiten Komponente relativ zu der ersten Komponente durch Einschränken der Drehbeweglichkeit des Lagerkörpers in der zweiten Komponente begrenzt werden.

Vorteilhaft ist es ferner, daß die Längsseite an einem Mittelstück des Lagerkörpers ausgebildet ist. Dadurch sind relativ lange Länsseiten an dem Lagerkörper gegeben, die eine vorteilhafte Führung des Lagerkörpers an seinem Mittelstück an den Längsflächen der Längskörper der zweiten Komponente gewährleisten.

Vorteilhaft ist es, daß die zweite Komponente eine mehrfach gekrümmte Fläche aufweist, die bei einer translativen Bewegung der zweiten Komponente relativ zu der ersten Komponente und oder einer Drehung des Führungselementes relativ zu der zweiten Komponente die Bewegung des Verriegelungskopfes begrenzt zum Begrenzen der Translativbewegung oder der Drehbewegung. Dadurch kann die translative Bewegung der zweiten Komponente relativ zu der ersten Komponente durch Anschlagen der zweiten Komponente an ihrer mehrfach gekrümmten Fläche, vorzugsweise an der Oberfläche des Verriegelungskopfes, begrenzt werden, wobei auch die Drehbewegung durch eine spezielle Ausgestaltung der mehrfach gekrümmten Fläche eingeschränkt werden kann.

In vorteilhafter Weise ist das Führungselement mit einem Verriegelungskopf verbunden, der zum Verbinden der ersten Komponente mit der zweiten Komponente in die zweite Komponente eingreift. Dadurch wird eine unmittelbare Verbindung zwischen der zweiten Komponente und dem Verriegelungskopf geschaffen, die insbesondere von der Lagerung des Lagerkörpers in der zweiten Komponente unabhängig ist. Dabei ist es möglich, daß der Verriegelungskopf in einer Stellung, in der seine Längsachse bezüglich der zweiten Komponente eine vorgegebene Drehstellung einnimmt, aus der zweiten Komponente auch wieder entfernbar ist.

Vorteilhaft ist es, daß der Verriegelungskopf in einen Sackraum der zweiten Komponente eingreift, wobei eine Entfernung des Verriegelungskopfes durch Anschlagen des Verriegelungskopfes an eine Anschlagfläche des Sackraums verhindert ist. Dadurch ist auch bei hohen Zugbelastungen, die auf die Gelenkprothese in einer Richtung, die senkrecht zu der Lagerfläche der zweiten Komponente ist, einwirken, eine Verbindung zwischen der ersten Komponente und der zweiten Komponente gewährleistet. Das Einbringes des Verriegelungskopfes kann dabei in einfacher Weise erfolgen, indem die Oberfläche des Verriegelungskopfes gekrümmt ausgebildet ist und oder eine oder mehrere Abschrägungen an der zweiten Komponente im Bereich des Sackraums vorgesehen sind.

Vorteilhaft ist es, daß die zweite Komponente ein Langloch aufweist, daß der Lagerkörper mit einem Stift verbunden ist, der in das Langloch eingreift, und daß die Drehung des Führungselementes relativ zu der zweiten Komponente durch Anschlagen der Zylindermantelfläche an den Anschlagflächen des Langlochs begrenzt ist. Dadurch kann die Drehung der zweiten Komponente relativ zu der ersten Komponente mittels der Zusammenwirkung zwischen dem Lagerkörper und der zweiten Komponente in einfacher Weise vorgebbar begrenzt werden, ohne daß die translative Beweglichkeit zwischen der zweiten Komponente und der ersten Komponente beeinflußt ist.

Vorteilhaft ist es ferner, daß das Langloch bezüglich der Mittelachse der zweiten Komponente radial gekrümmt ist, wobei die Mittelachse der zweiten Komponente im montierten Zustand der Gelenkprothese zumindes im Wesentlichen mit der Drehachse des Lagerkörpers übereinstimmt. Dadurch kann die äußere und die innere Führungsfläche des gekrümmten Langlochs zur Führung des Lagerkörpers mittels des Stifes verbessert werden. Außerdem ist dadurch die Geometrie des Langlochs an die Drehbewegung des mit dem Lagerkörper verbundenen Stiftes angepaßt.

In vorteilhafter Weise ist die Oberfläche des Verriegelungskopfes gekrümmt ausgebildet, wobei die Krümmung der Oberfläche an die Krümmung einer mehrfach gekrümmten Fläche der zweiten Komponente, die den Verriegelungskopf zumindest im Wesentlichen umgibt, angepaßt ist. Dadurch kann zum einen eine Führung des Verriegelungskopfes an der mehrfach gekrümmten Fläche der zweiten Komponente erfolgen und zum anderen kann die Beweglichkeit des Verriegelungskopfes zum Begrenzen der translativen Bewegung und oder der Drehbewegung der zweiten Komponente bezüglich der ersten Komponente begrenzt werden.

Vorteilhaft ist es, daß der Verriegelungskopf zumindest eine Seitenfläche aufweist, die in einer vorgegebenen Drehstellung des Verriegelungskopf mit einer Innenfläche des Lagerkörpers fluchtet zum Lösen des Eingriffs des Verriegelungskopf in den Lagerkörper. Dadurch lässt sich die Verriegelung entriegeln, um die Demontage der Gelenkprothese zu vereinfachen.

Die Erfindung wird nachfolgend anhand von Ausführungsbeispielen unter Bezugnahme auf die Zeichnung näher beschrieben. In der Zeichnung zeigen:
- Fig. 1: eine erste Komponente und ein Führungselement einer Gelenkprothese gemäß einem ersten Ausführungsbeispiel;
- Fig. 2A: eine zweite Komponente und einen Lagerkörper der Gelenkprothese gemäß dem ersten Ausführungsbeispiel;
- Fig. 2B: die zweite Komponente und den Lagerkörper, die in Fig. 2A gezeigt sind, worin der Lagerkörper in einer alternativen Drehstellung eingestellt ist;
- Fig. 3: einen Längsschnitt durch die in Fig. 2B dargestellte Gelenkprothese;
- Fig. 4A: einen Lagerkörper gemäß einem zweiten Ausführungsbeispiel;
- Fig. 4B: einen Lagerkörper gemäß einem dritten Ausführungsbeispiel;
- Fig. 4C: einen Lagerkörper gemäß einem vierten Ausführungsbeispiel;
- Fig. 5A: ein Führungselement gemäß einem zweiten Ausführungsbeispiel;
- Fig. 5B: ein Führungselement gemäß einem dritten Ausführungsbeispiel;
- Fig. 5C: ein Führungselement gemäß einem vierten Ausführungsbeispiel;
- Fig. 6: einen Längsschnitt durch die in Fig. 1 teilweise dargestellte Gelenkprothese;
- Fig. 7A: eine erste Komponente und ein Führungselement einer Gelenkprothese gemäß einem fünften Ausführungsbeispiel;
- Fig. 7B: die in Fig. 7A dargestellte erste Komponente und das Führungselement gemäß dem fünften Ausführungsbeispiel in einer perspektivischen Darstellung;
- Fig. 8A: einen Lagerkörper gemäß einem fünften Ausführungsbeispiel;
- Fig. 8B: den Lagerkörper gemäß dem fünften Ausführungsbeispiel in der Unteransicht;
- Fig. 9A: eine zweite Komponente gemäß einem fünften Ausführungsbeispiel;
- Fig. 9B: die in Fig. 9A dargestellte zweite Komponente gemäß dem fünften Ausführungsbeispiel in einer perspektivischen Darstellung;
- Fig. 10: eine Gelenkprothese gemäß dem fünften Ausführungsbeispiel, wobei die zweite Komponente geschnitten dargestellt ist;
- Fig. 11A: einen Lagerkörper gemäß einem sechsten Ausführungsbeispiel;
- Fig. 11B: den Lagerkörper gemäß dem sechsten Ausführungsbeispiel in einer Unteransicht;
- Fig. 12A: eine zweite Komponente der Gelenkprothese gemäß einem sechsten Ausführungsbeispiel;
- Fig. 12B: die zweite Komponente der Gelenkprothese gemäß dem sechsten Ausführungsbeispiel in einer Schnittdarstellung entlag der in Fig. 12A mit 12B bezeichneten Schnittlinie; und
- Fig. 13: eine Gelenkprothese im montierten Zustand gemäß einem sechsten Ausführungsbeispiel, die die erste Komponente gemäß dem fünften Ausführungsbeispiel, den Lagerkörper gemäß dem sechsten Ausführungsbeispiel und die zweite Komponente gemäß dem sechsten Ausführungsbeispiel umfaßt, wobei die zweite Komponente geschnitten dargestellt ist.

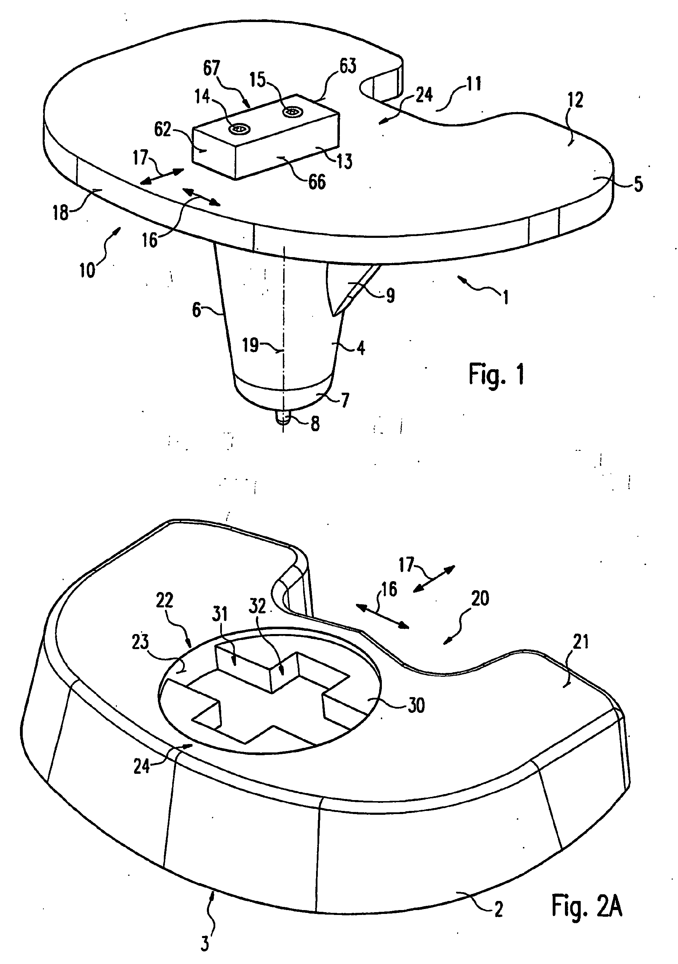

Fig. 1 und 2A zeigen eine erste Komponente 1 und eine zweite Komponente 2, die zusammen die Grundbestandteile einer Gelenkprothese bilden. Die erste Komponente 1 der Gelenkprothese ist zumindest mittelbar mit einem ersten Knochen verbunden, beispielsweise indem der erste Knochen auf einer bestimmten Höhe abgetrennt, gegebenenfalls ausgeschabt und die ersten Komponente 1 in den entstandenen Hohlraum eingebracht und verkeilt wird. Die zweite Komponente 2 weist auf ihrer Oberseite 3, die in der Fig. 2A nicht dargestellt ist, eine oder mehrere Lagerschalen auf, an denen sich ein zweiter Knochen, z.B. mittels einer oder mehrerer Abstützmittel, abstützt. Die Gelenkprothese dient insbesondere als Kniegelenkprothese, d.h. zum Ausbilden einer Gelenkverbindung zwischen Tibia als ersten Knochen und Femur als zweiten Knochen. Die Gelenkprothese eignet sich allerdings auch für andere Anwendungsfälle.

Fig. 1 zeigt die erste Komponente 1 der Gelenkprothese, die einen Befestigungszapfen 4 und eine Lagerplatte 5 umfaßt. Der Befestigungszapfen 4 weist einen konischen Abschnitt 6, eine abgerundete Kappe 7 und einen Dornfortsatz 8 auf, um das Einbringen des Befestigungszapfens 4 der ersten Komponente 1 in den ersten Knochen zu erleichtern. Um die Verkeilung der ersten Komponente in dem ersten Knochen zu verbessern, sind zwei keilförmige, flügelartige Befestigungsmittel 9, 10 vorgesehen, die gegenüberliegend zueinander angeordnet sind. In der Fig. 1 ist nur das Befestigungsmittel 9 dargestellt, das Befestigungsmittel 10 ist aufgrund der perspektivischen Ansicht von der Lagerplatte 5 verdeckt. Die Lagerplatte 5 weist eine Aussparung 11 auf, die für Bänder und dergleichen vorgesehen ist. Bei Verwendung der Gelenkprothese als Kniegelenkprothese ist die Aussparung 11 dorsal angeordnet, um das hintere Kreuzband des Kniegelenks aufzunehmen.

Die Lagerplatte 5 weist außerdem eine Lagerfläche 12 auf, auf der die zweite Komponente 2 im montierten Zustand der Gelenkprothese gelagert ist. Auf der Lagerfläche 12 ist ein Führungselement 13 befestigt, das in diesem Ausführungsbeispiel eine quaderförmige Form hat. Die Befestigung des Führungselements 13 an der ersten Komponente 1 erfolgt mittels Schrauben 14, 15, die als Imbusschrauben ausgeführt sind. Das Führungselement 13 kann dabei bezüglich einer Querrichtung 16 der Lagerfläche 12 der ersten Komponente 1 zumindest im wesentlichen mittig, d.h. zentriert, auf der Lagerfläche 12 angeordnet. Es ist auch denkbar das Führungselement 13 exzentrisch Richtung medial anzuordnen. Bezüglich einer Längsrichtung 17 der Lagerfläche 12 ist das Führungselement 13 zur Vorderseite 18 der ersten Komponente 1 hin versetzt zu der Achse 19 des Befestigungszapfens 4, die im wesentlichen mit der Mittelachse des ersten Knochens übereinstimmt, angeordnet. Dies ist in Bezug auf die bei der zweiten Komponente 2 in Übereinstimmung mit der Aussparung 11 vorgesehenen Aussparung 20, die in Fig. 2A dargestellt ist, von besonderem Vorteil, wie es anhand der Fig. 2A detailliert beschrieben ist.

Anstelle der Schrauben 14, 15, die das Führungselement 13 mit der ersten Komponente 1 verbinden, können auch Nieten oder dergleichen vorgesehen sein. Außerdem ist es möglich, das Führungselement 13 und die erste Komponente 1 einstückig auszubilden.

Die erste Komponente 1 und das Führungselement 13 können aus dem gleichen Material, insbesondere einem Metall, wie Kobalt-Chrom-Legierung oder Titan, ausgebildet sein.

Die in Fig. 2A dargestellte zweite Komponente 2 weist eine Lagerfläche 21 auf, die im montierten Zustand der Gelenkprothese zumindest teilweise an der Lagerfläche 12 der ersten Komponente anliegt. Hierfür sind die Lagerfläche 12 der ersten Komponente 1 und die Lagerfläche 21 der zweiten Komponente 2 eben ausgeführt. Die zweite Komponente 2 weist eine zylinderförmig ausgebildete Aussparung 22 auf, die an die Lagerfläche 21 angrenzt. Aufgrund der zylinderförmigen Aussparung 22 ist an der zweiten Komponente 2 eine zylindermantelförmige Fläche 23 ausgebildet.

In dem beschriebenen ersten Ausführungsbeispiel ist die Lagerfläche 21 der zweiten Komponente 2 flächenmäßig kleiner als die Lagerfläche 12 der ersten Komponente 1. Hierdurch ist gewährleistet, daß die zweite Komponente 2 bei kleinen Auslenkungen aus ihrer Normalposition höchstens geringfügig über die Lagerfläche 12 der ersten Komponente 1 hinausragt. Dementsprechend ist die der Aussparung 11 der ersten Komponente 1 gegenüberliegende Aussparung 20 der zweiten Komponente 2 in der Längsrichtung 17 zumindest im wesentlichen gleich tief und in Querrichtung 16 breiter ausgebildet. Da die Lagerfläche 21 im mittleren Bereich 24 in Längsrichtung 17 schmäler als die Lagerfläche 12 ausgebildet ist, ist für die Gewährleistung eines maximalen Verschiebebereichs in Längsrichtung 17 das Führungselement 13 in der oben beschriebenen Weise versetzt zu der Achse 19 anzuordnen. Die Neutralposition der zweiten Komponente 2 ist dann bezüglich der Gesamtfläche der Lagerfläche 12 betrachtet zur Vorderseite 18 hin verschoben, aber bezüglich des mittleren Bereichs 24 zumindest im wesentlichen zentriert.

In der zylinderförmigen Aussparung 22 ist ein Lagerkörper 30 gelagert. Der Lagerkörper 30 weist eine erste Aussparung 31 und eine zweite Aussparung 32 auf, die miteinander verbunden und zur Seite der Lagerfläche 21, d.h. im montierten Zustand der Gelenkprothese zur Seite der Lagerfläche 12 der ersten Komponente 1 offen sind. Die Aussparungen 31, 32 sind dabei nutförmig ausgebildet. In der in Fig. 2A dargestellten Drehstellung des Lagerkörpers 30 ist die erste Aussparung 31 in Querrichtung 16 orientiert, während die zweite Aussparung 32 senkrecht zur ersten Aussparung 31, d.h. in Längsrichtung 17, orientiert ist. Die Aussparungen 31, 32 überlagern sich dadurch zu einer kreuzförmigen Aussparung in dem Lagerkörper 30. Die Länge der zweiten Aussparung 32 in Längsrichtung 17 ist im wesentlichen gleich der Länge des Führungselements 13 in Längsrichtung 17, so daß das in die zweite Aussparung 32 im montierten Zustand der Gelenkprothese eingebrachte Führungselement 13 in dem Lagerkörper 30 gegenüber Bewegungen in Querrichtung 16 und Längsrichtung 17 fixiert ist. Dadurch greift das Führungselement 13 mittels des Lagerkörpers 30 in die zweite Komponente 2 ein.

Der Lagerkörper 30 ist in der zylinderförmigen Aussparung 22 drehbar gelagert, so daß der Lagerkörper 30 eine Drehung des Führungselements 13 relativ zu der zweiten Komponente 2 ermöglicht, wodurch die zweite Komponente 2 gegenüber der ersten Komponente 1 der Gelenkprothese drehbar ist.

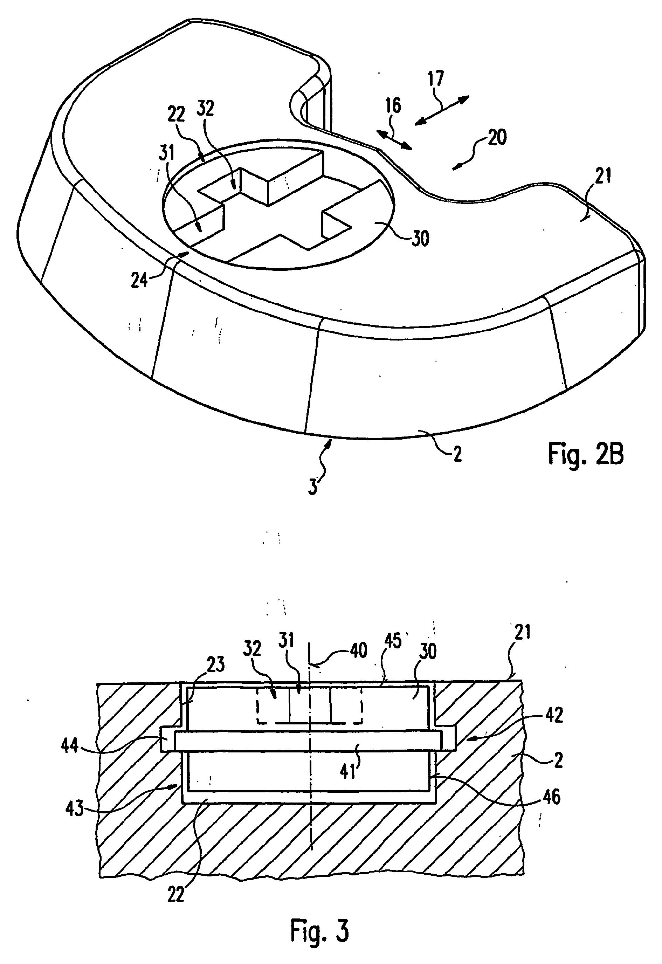

Fig. 2B zeigt die in Fig. 2A dargestellte zweite Komponente 2, bei der der Lagerkörper 30 in einer gegenüber der in Fig. 2A dargestellten Stellung um 90° gedrehten Stellung angeordnet ist. Bereits beschriebene Elemente sind mit übereinstimmenden Bezugszeichen versehen, wodurch sich eine wiederholende Beschreibung erübrigt.

In der in Fig. 2B dargestellten Drehstellung des Lagerkörpers 30 ist die erste Aussparung 31 entlang der Längsrichtung 17 orientiert und die zweite Aussparung 32 ist entlang der Querrichtung 16 orientiert. Wird das Führungselement 13 zum Montieren der Gelenkprothese in die erste Aussparung 31 eingebracht, dann kann das Führungselement 13 in der ersten Aussparung 31 entlang der Längsrichtung 17 translativ bewegt werden, da die Länge des Führungselements 13 entlang der Längsrichtung 17 kleiner ist als die Länge der ersten Aussparung 31. Die Drehbarkeit des Führungselements 13 relativ zu der zweiten Komponente 2 der Gelenkprothese bleibt dabei erhalten. Da die Drehachse durch das durch den Lagerkörper 30 und die zylindrische Aussparung 22 gebildete Drehlager definiert ist, das auf seiten der zweiten Komponente 2 vorgesehen ist, ist unabhängig von der Stellung des Führungselements 13 in der ersten Aussparung 31 die Drehbarkeit bezüglich einer relativ zu der zweiten Komponente 2 feststehenden Drehachse gegeben. Dadurch kann der anatomische Bewegungsablauf eines menschlichen oder tierischen Gelenks besonders vorteilhaft nachgebildet werden, da der mittels geeigneter Abstützmittel in den Lagerschalen der zweiten Komponente 2 abgestützte zweite Knochen beim Drehen der zweiten Komponente relativ zu der ersten Komponente bezüglich der Achse 19, die im wesentlichen mit der Mittelachse des ersten Knochens übereinstimmt, in Querrichtung 16 im wesentlichen nicht verschwenkt wird.

Die translative Bewegung der zweiten Komponente 2 zu der ersten Komponente 1 ist durch Anschlagen des Führungselements 13 an der zylindermantelförmigen Fläche 23 der zylinderförmigen Aussparung 22 begrenzt. Die Beweglichkeit ist dabei unabhängig von der Drehwinkelstellung des Lagerkörpers 30 in der zylinderförmigen Aussparung 22 der zweiten Komponente 2.

Fig. 3 zeigt einen auszugsweisen Schnitt durch die in Fig. 2B dargestellte zweite Komponente 2 durch die Drehachse 40 des Lagerkörpers 30. Bereits beschriebene Elemente sind mit übereinstimmenden Bezugszeichen versehen, wodurch sich eine wiederholende Beschreibung erübrigt.

Der Lagerkörper 30 weist einen umlaufenden Kreisbund 41 auf, der in diesem Ausführungsbeispiel in einem mittleren Bereich 42 des Lagerkörpers 30 ausgebildet ist. Der umlaufende Kreisbund 41 kann auch auf einer anderen Höhe an dem Lagerkörper 30, z.B. im Bereich 43 des unteren Endes des Lagerkörpers 30, vorgesehen sein. Der umlaufende Kreisbund 41 greift in eine umfänglich ausgebildete Nut 44 der zweiten Komponente 2 ein. Die Nut 44 ist mit der zylinderförmigen Aussparung 22 der zweiten Komponente 2 verbunden, so daß die zylindermantelförmige Fläche 23 an der Nut 44 unterbrochen ist. Der Lagerkörper 30 wird in die zylinderförmige Aussparung 22 eingepreßt, so daß eine unlösbare, aber spielbehaftete Verbindung zwischen dem Lagerkörper 30 und der zweiten Komponente 2 besteht. Die Stirnseite 45 des Lagerkörpers 30 ist gegenüber der Lagerfläche 21 der zweiten Komponente 2 zurückgesetzt, so daß die erste Komponente 1 die Anlagekraft vollständig über die Lagerfläche 21 an die zweite Komponente 2 übermittelt. Daher hat die aus dem umlaufenden Kreisbund 41 und der umlaufenden Nut 44 bestehende Verbindung lediglich die Aufgabe, den Lagerkörper 30 in der zylinderförmigen Aussparung 22 zu halten.

Die über das in eine der Aussparungen 31, 32 eingreifende Führungselement 13 auf den Lagerkörper 30 übertragene Gelenkkraft wird an der zylindermantelförmigen Fläche 23 der zweiten Komponente 2 mittels der Mantelfläche 46, die durch den umlaufenden Bund 41 unterbrochen ist, übertragen.

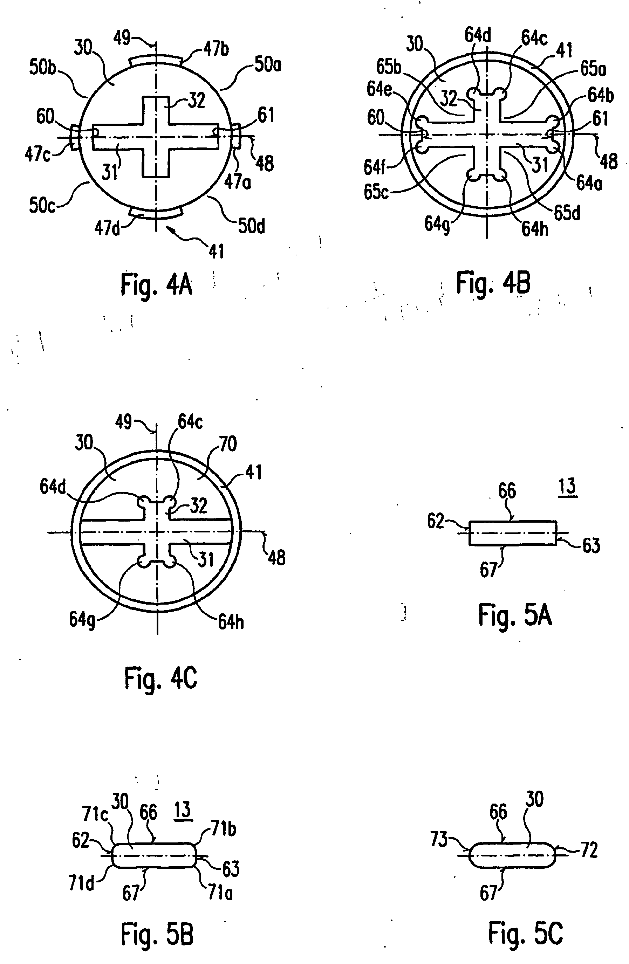

Fig. 4A zeigt einen Lagerkörper 30 gemäß einem zweiten Ausführungsbeispiel. In diesem Ausführungsbeispiel ist der Bund 41 als abschnittsweise umlaufender Kreisbund ausgebildet, wobei er aus den Kreisbundsegmenten 47a bis 47d besteht. Die Kreisbundsegmente 47a und 47c sind in der Verlängerung der ersten Aussparung 31 vorgesehen und bezüglich der Längsachse 48 der ersten Aussparung symmetrisch ausgebildet. Die Kreisbundsegmente 47b und 47d sind in der Verlängerung der zweiten Aussparung 32 vorgesehen und symmetrisch zur Längsachse 49 der zweiten Aussparung 32 ausgebildet. In den Bereichen 50a bis 50d, in denen der Bund 41 unterbrochen ist, sind im montierten Zustand des Lagerkörpers 30 Unterbrechungen der kreisringförmigen Nut 44 ausgebildet, an denen die Kreisbundsegmente 47a bis 47d bei einer bestimmten Drehwinkelstellung anschlagen. Auf diese Weise kann der Bereich vorgegeben werden, in dem eine Drehung des Lagerkörpers 30 relativ zu der zweiten Komponente 2 möglich ist, wodurch die Drehbeweglichkeit der Gelenkprothese vorgebbar ist. Die Drehbeweglichkeit kann dabei auch asymmetrisch bezüglich der in Längsrichtung 17 ausgerichteten Normalstellung des Gelenks sein, z.B. indem die Kreisbundsegmente 47a, 47c asymmetrisch bezüglich der Längsachse 48 oder die Kreisbundsegmente 47b, 47d asymmetrisch bezüglich der Längsachse 49 ausgebildet sind.

Wenn das Führungselement 13 im montierten Zustand der Gelenkprothese in die erste Aussparung 31 eingreift, dann wird die translative Bewegung der zweiten Komponente relativ zu der ersten Komponente durch Anschlagen des Führungselements 13 an den Anschlagflächen 60, 61 der Aussparung 31 begrenzt. Im Vergleich zum ersten Ausführungsbeispiel, das in der Fig. 2B dargestellt ist, erfolgt die Begrenzung der Translationsbewegung durch den Lagerkörper 30 und nicht durch die zylindermantelförmige Fläche 23 der zylinderförmigen Aussparung 22 der zweiten Komponente 2. Da die Anschlagflächen 60, 61 im Gegensatz zur gekrümmten zylindermantelförmigen Fläche 23 eben sind, erfolgt die Abstützung dadurch gleichmäßig über die Stirnfläche 62 bzw. 63 des Führungselements 13 gemäß dem in Fig. 1 dargestellten ersten Ausführungsbeispiel.

Fig. 4B zeigt einen Lagerkörper 30 gemäß einem dritten Ausführungsbeispiel. In diesem Ausführungsbeispiel weist der Lagerkörper 30 einen umlaufenden Kreisbund 41 auf. Außerdem sind eine erste 31 und eine zweite Aussparung 32 vorgesehen, die gegenüber dem in der Fig. 4A dargestellten zweiten Ausführungsbeispiel zusätzlich um als Bohrungen ausgebildete Kantenausformungen 64a bis 64h erweitert sind. Dabei sind die Kantenausformungen 64a bis 64h in den Kanten der Aussparungen 31, 32 angeordnet, wobei im Bereich der Innenkanten 65a bis 65d keine Kantenausformungen vorgesehen sind. Durch die Kantenausformungen 64a, 64b, 64e, 64f ist gewährleistet, daß das Führungselement 13 bei einer Bewegung in der ersten Aussparung 31 entlang der Längsachse 48 vollständig an den Anschlagflächen 60, 61 und nicht nur in den Kantenbereichen anschlägt. Da das Führungselement 13 in jeder Stellung, also auch in den Stellungen, in denen die Stirnflächen 62, 63 an den Anschlagflächen 60 bzw. 61 anliegen, mit den längsseitigen Seitenflächen 66, 67 neben den Innenkanten 65a bis 65d liegt, so daß diese von den Stirnflächen 62, 63 nicht überschritten werden, ist eine Abrundung dieser nicht erforderlich. Eine Abrundung der Innenkanten 65a bis 65d kann allerdings dennoch, speziell für ein anders, insbesondere in Längsrichtung 17 kürzer, ausgebildetes Führungselement 13, sinnvoll sein.

Die Kantenausformungen 64c, 64d, 64g, 64h der zweiten Aussparung 32 erleichtern das Einbringen des Führungselements 13, so daß insbesondere gewährleistet ist, daß die Seitenflächen 62, 63, 66, 67 an den entsprechenden Seitenflächen der zweiten Aussparung 32 im wesentlichen spielfrei anliegen.

Fig. 4C zeigt einen Lagerkörper 30 gemäß einem vierten Ausführungsbeispiel. Der Lagerkörper 30 weist einen umlaufenden Kreisbund 41 auf, der mit dem zylinderförmigen Grundkörper 70 des Lagerkörpers 30 verbunden ist. Der Grundkörper 70 des Lagerkörpers 30 weist eine als durchgehende Nut ausgebildete erste Aussparung 31 auf, in der das Führungselement 13 entlang der Längsachse 48 geführt ist, wobei die translative Bewegung des Führungselements 13 nur durch die zylindermantelförmige Fläche 23 der zylinderförmigen Aussparung 22 der zweiten Komponente 2 der Gelenkprothese begrenzt ist. Ferner weist der Grundkörper 70 eine zweite Aussparung 32 auf, die ebenfalls nutförmig ausgebildet ist. Die Längsachse 49 der zweiten Aussparung 32 ist dabei senkrecht zur Längsachse 48 der ersten Aussparung 31 orientiert. Im Bereich der Kanten der zweiten Aussparung 32 sind Kantenausformungen 64c, 64d, 64g, 64h vorgesehen, die als Bohrungen ausgeführt sind, wobei die Bohrungen zumindest so tief wie die Aussparung 32 ausgebildet sind.

Fig. 5A zeigt den Querschnitt des Führungselements 13 gemäß einem zweiten Ausführungsbeispiel. Das Führungselement 13 weist Stirnseiten 62, 63 und Seitenflächen 66, 67 auf, an denen das Führungselement 13 in der ersten Aussparung 31 geführt ist. Das Führungselement 13 ist einstückig mit der Lagerplatte 5 der ersten Komponente verbunden, so daß keine Durchgangsbohrungen für Schrauben 14, 15 oder dergleichen erforderlich sind.

Fig. 5B zeigt das in Fig. 5A dargestellte Führungselement 13 im Querschnitt gemäß einem dritten Ausführungsbeispiel. Das Führungselement 13 weist abgerundete Kanten 71a bis 71d auf. Dadurch eignet sich das Führungselement 13 im besonderen in Zusammenwirkung mit dem Lagerkörper 30 gemäß dem zweiten Ausführungsbeispiel, der in Fig. 4A dargestellt ist, da abgerundete Kanten 64a bis 64h der Aussparungen 31, 32, wie sie in Fig. 4B dargestellt sind, aufgrund der abgerundeten Kanten 71a bis 71d des Führungselements 13 nicht erforderlich sind. Durch die abgerundeten Kanten 71a bis 71d des Lagerkörpers 30 ist gewährleistet, daß der Lagerkörper 30 beim Anschlagen an den Anschlagflächen 60, 61 der ersten Aussparung 31 gleichmäßig an der Fläche 62 bzw. 63 anliegt. Das Lagerelement 30 gemäß dem dritten Ausführungsbeispiel eignet sich auch in Zusammenwirkung mit einer durchgehenden ersten Aussparung 31, wie sie z.B. in Fig. 4C dargestellt ist, da durch die abgerundeten Kanten 71a bis 71d eine Beschädigung der zylindermantelförmigen Fläche 23 der zylinderförmigen Aussparung 22 der zweiten Komponente 2 vermieden wird.

Fig*.* 5C zeigt das in Fig. 5A dargestellte Führungselement 13 gemäß einem vierten Ausführungsbeispiel. Der Lagerkörper 30 gemäß dem vierten Ausführungsbeispiel weist Seitenflächen 66, 67 und abgerundete Enden 72, 73 auf. Stirnflächen 62, 63, wie sie gemäß dem ersten bis dritten Ausführungsbeispiel vorgesehen sind, sind durch die abgerundeten Enden 72, 73 gegeben. Vorteilhaft ist es, daß die abgerundeten Enden 72, 73 zumindest abschnittsweise den gleichen Krümmungsradius wie die zylindermantelförmige Fläche 23 der zylinderförmigen Aussparung 22 aufweisen, falls das Führungselement 13 in Verbindung mit einer durchgehenden ersten Aussparung 31, wie sie z.B. in Fig. 4C dargestellt ist, zum Einsatz kommt, da dann eine vorteilhafte, großflächige Anlage an der zylindermantelförmigen Fläche 23 beim Anschlagen des Führungselements 13 an dieser gegeben ist.

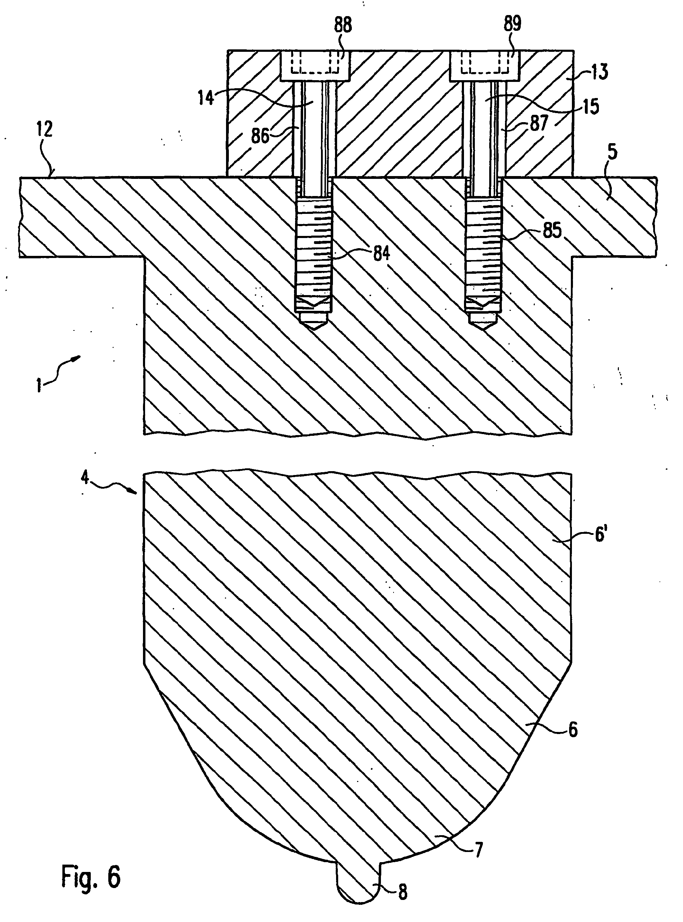

Fig. 6 zeigt einen Längsschnitt durch die in Fig. 1 dargestellte erste Komponente 1 gemäß einem fünften Ausführungsbeispiel. Bei diesem Ausführungsbeispiel weist die erste Komponente einen Lagerzapfen 4 auf, der einen zylinderförmigen Abschnitt 6', einen konischen Abschnitt 6 und eine abgerundete Kappe 7 umfaßt. Ferner ist an der abgerundeten Kappe 7 ein Dornfortsatz 8 vorgesehen. Der zylinderförmige Abschnitt 6' ist mit der Lagerplatte 5 der ersten Komponente 1 verbunden, die eine Lagerfläche 12 umfaßt. Das Führungselement 13 ist mittels Schrauben 14, 15 in die erste Komponente 1 der Gelenkprothese eingeschraubt, wobei die Gewindeabschnitte 84, 85 der Schrauben 14, 15 sowohl in die Lagerplatte 5 als auch in den Befestigungszapfen 4 eingreifen. Die Schrauben 14, 15 erstrecken sich dabei durch in dem Führungselement 13 ausgebildete Bohrungen 86, 87. Zum Einschrauben der Schrauben 14, 15 weisen diese Imbusköpfe 88, 89 auf.

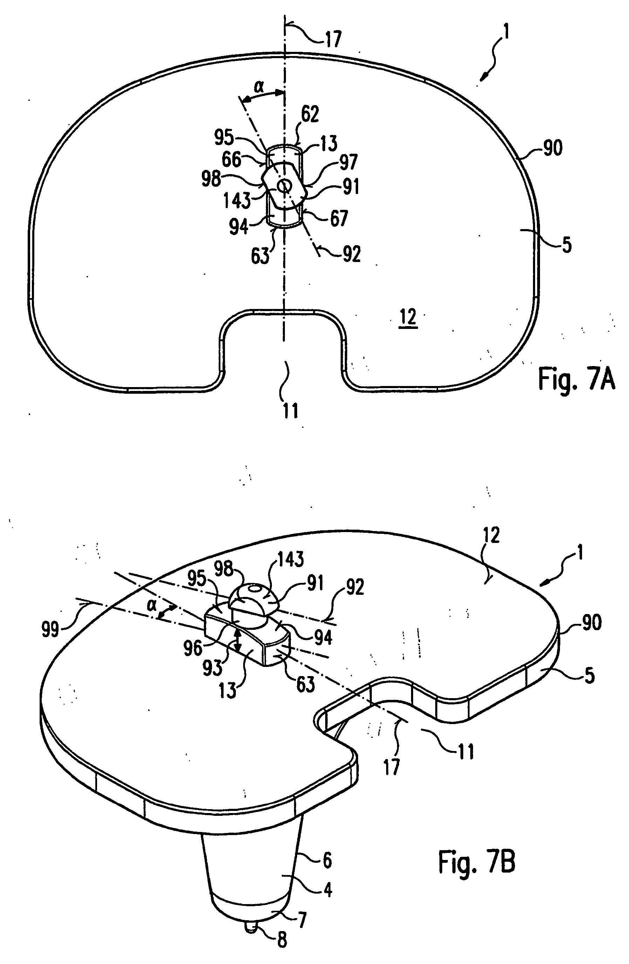

Fig. 7A zeigt eine erste Komponente 1 und ein Führungselement 13 einer Gelenkprothese gemäß einem fünften Ausführungsbeispiel. Bereits beschriebene Elemente sind in dieser und in allen anderen Figuren mit übereinstimmenden Bezugszeichen versehen, wodurch sich eine wiederholende Beschreibung erübrigt.

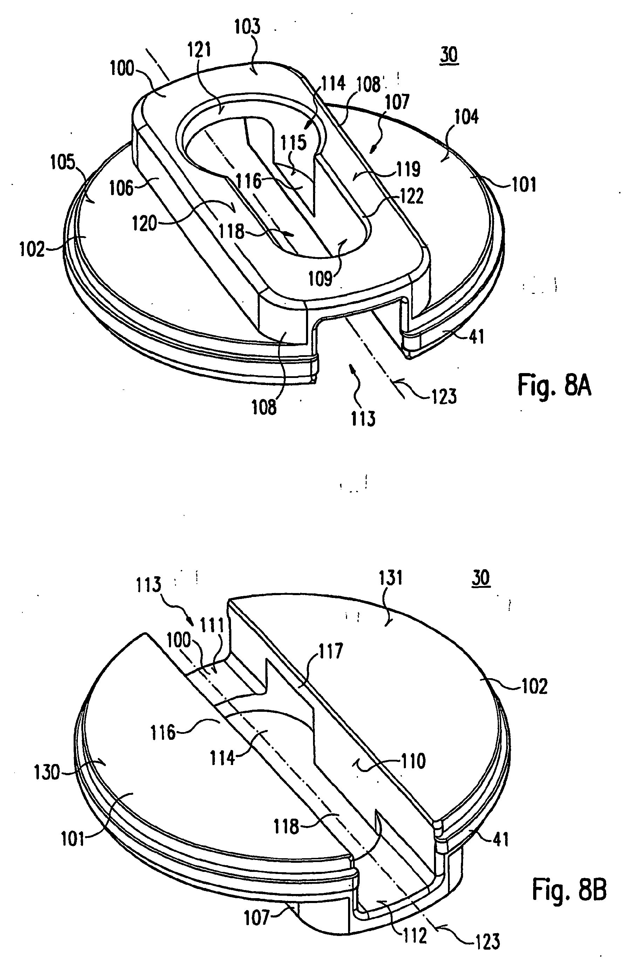

Die erste Komponente 1 weist eine Lagerplatte 5 auf, in der eine Aussparung 11 für Bänder oder dergleichen vorgesehen ist. Die Lagerplatte 5 weist ferner einen umlaufenden Rand 90 auf, der abgerundet ausgebildet ist, um ein vorteilhaftes Gleiten der zweiten Komponente 2 auf der Lagerfläche 12 der Lagerplatte 5 der ersten Komponente 1 auch für den Fall zu gewährleisten, daß die zweite Komponente 2 teilweise über die Lagerfläche 12 hinaussteht. Das Führungselement 13 ist mit einem Verriegelungskopf 91 verbunden, der zum Verriegeln des Eingriffs des Führungselemente 13 in die zweite Komponente 2 dient, wie es an Hand der Fig. 8A fortvolgende noch näher beschrieben ist. Die Längsachse 92 des Verriegelungskopfes 91 ist gegenüber der Längsrichtung 17 um den Schwenkwinkel α verschwenkt, wobei die Verschwenkung in einer Ebenen, die parallel zu der Lagerfläche 12 ist, erfolgt. In dem beschriebenen fünften Ausführungsbeispiel beträgt die Größe des Schwenkwinkels α zumindest näherungsweise 15°. Die Verschwenkung kann jedoch auch um einen anderen Schwenkwinkel α, insbesondere um -15°, d. h. in anderer Richtung, oder um 90°, erfolgen.

Fig. 7B zeigt die in Fig. 7A dargestellte erste Komponente 1 und das Führungselement 13 gemäß dem fünften Ausführungsbeispiel in einer perspektivischen Darstellung.

Das Führungselement 13 gemäß dem fünften Ausführungsbeispiel weist Stirnflächen 62, 63 auf, von denen in der Fig. 7B nur die Stirnfläche 63 dargestellt ist. Von der Stirnfläche 63 aus nimmt die Höhe des Führungselements 13 bis zur Mitte des Führungselements 13 zu, wo sie eine maximale Höhe 93 erreicht. Von der maximalen Höhe 93 aus nimmt die Höhe des Führungselements 13 in Richtung auf die Stirnfläche 62 wieder ab, wobei die Höhe des Führungselements 13 auf der Seite der Stirnfläche 62 zumindest im Wesentlichen gleich der Höhe auf der Seite der Stirnfläche 63 ist. Bei dem beschriebenen fünften Ausführungsbeispiel ergibt sich dadurch ein zumindest im Wesentlichen symmetrischer Aufbau des Führungselements 13. Bei diesem sind die beiden, auf der Oberseite des Führungselements 13 ausgebildeten Flächen 94, 95 entgegengesetzt zueinander gegenüber der Lagerfläche 12 der Lagerplatte 5 der ersten Komponente geneigt angeordnet. In der Mitte des Führungselements 13, d. h. im Bereich der maximalen Höhe 93, ist an dem Führungselement 13 ein zumindest im Wesentlichen zylinderförmiger Verbindungszapfen 96 vorgesehen, der den Verriegelungskopf 91 mit dem Führungselement 13 verbindet.

Der Gegenstand, der sie aus dem Verriegelungskopf 91 und dem Verbindungszapfen 96 ergibt, ist zumindest im Wesentlichen pilzförmig ausgebildet, wobei längsseits bezüglich der Längsachse 92 Seitenflächen 97, 98 an dem Verriegelungskopf 91 vorgesehen sind, die senkrecht zu der Lagerfläche 12 der Lagerplatte 5 orientiert sind.

In der Fig. 7B ist außerdem die Projektion der Längsachse 92 des Verriegelungskopfes 91 in eine Ebene, die parallel zu der Lagerfläche 12 der Lagerplatte 5 der erste Komponente orientiert ist und in der die Achse liegt, die die Längsrichtung 17 angibt, dargestellt, wobei sich durch die Projektion die Achse 99 ergibt. Der Schwenkwinkel α, um den die Längsachse 92 des Verriegelungskopfes 91 gegenüber der Längsachse 17 des Führungselements 13 parallel zu der Lagerfläche 12 der Lagerplatte 5 verschwenkt ist, ergibt sich dann als Winkel zwischen der Längsachse 17 und der Achse 99.

Figuren 8A und 8B zeigen perspektivische Darstellungen eines Lagerkörpers 30 gemäß einem fünften Ausführungsbeispiel, der insbesondere für die Zusammenwirkung mit der ersten Komponente 1 und dem Führungselement 13 gemäß dem in den Fig. 7A und 7B gezeigten fünften Ausführungsbeispiel dient.

Der Lagerkörper 30 weist ein Mittelstück 100, ein ersten Seitenstück 101 und ein zweites Seitenstück 102 auf. Das Mittelstück 100 weist eine Oberseite 103 auf, das erste Seitenstück 101 weist eine Oberseite 104 auf und das zweite Seitenstück 102 weist eine Oberseite 105 auf. Das Mittelstück 100 weist außerdem Längsseiten 106, 107 auf, die mittels eines abgerundeten umlaufenden Randes 108 ineinander übergehen. Der Lagerkörper 30 weist außerdem eine erste Innenfläche 109 und eine zweite Innenfläche 110 auf, wobei die erste Innenfläch 109 im Ausführungsbeispiel sowohl an dem Mittelstück 100 als auch an dem ersten Seitenstück 101 ausgebildet ist und die zweite Innenfläche 110 sowohl an dem Mittelstück 100 als auch an dem zweiten Seitenstück 102 ausgebildet ist.

Die erste Innenfläche 109 und die zweite Innenfläche 110 sind gegenüberliegend zueinander angeordnet und durch zwei im Wesentlichen senkrecht zu den Innenflächen 109, 110 angeordnete Flächen 111, 112 miteinander verbunden. Die Flächen 111, 112, die erste Innenfläche 109 und die zweite Innenfläche 110 begrenzen eine nutförmige Aussparung 113 des Lagerkörpers 30. Die nutförmige Aussparung 113 ist mit einer zylinderförmigen Aussparung 114 verbunden, wobei die zylinderförmige Aussparung 114 an einer Stirnfläche 115 endet, die einen Absatz 116 an dem ersten Seitenstück 101 bildet. Gegenüberliegend zu der Stirnfläche 115, die an dem ersten Seitstück 101 ausgebildet ist, ist eine weitere Stirnfläche vorgesehen, die an dem zweiten Seitenstück 102 ausgebildet ist, wodurch ein Absatz 117 an dem zweiten Seitenstück 102 gegeben ist.

Ferner ist eine als Langloch ausgebildete Aussparung 118 in dem Mittelstück 100 vorgesehen, die teilweise mit der zylinderförmigen Aussparung 114 und teilweise mit der nutförmigen Aussparung 113 übereinstimmt. Durch die zylinderförmige Aussparung 114 und die als Langloch ausgebildete Aussparung 118 ist eine gemeinsame Aussparung gegeben, die die Form eines Schlüsselloches hat.

Da der Durchmesser der zylinderförmigen Aussparung 114 größer ist als die Breite der als Langloch ausgebildeten Aussparung 118, ist seitlich des Langlochs 118 an der Oberseite 103 des Mittelstücks 100 eine erste Führungsfläche 119 und eine zweite Führungsfläche 120 gegeben. Die Führungsflächen 119, 120 dienen, wie es an anderer Stelle noch genauer erläutert ist, insbesondere zum Führen des Verriegelungskopfes 91 im verriegelten Zustand der Gelenkprothese.

An dem Lagerkörper 30 ist ein umlaufender Kreisbund 41 vorgesehen, der allerdings durch die nutförmige Aussparung 113 an Stellen im Bereich der Flächen 111, 112 unterbrochen ist. Die Kreisbund 41 ist im Wesentlichen in der Mitte der Höhe der beiden Seitenstücke 101, 102 angeordnet.

Das Mittelstück 100 des Lagerkörpers 30 weist durch die zylinderförmige Aussparung 114 und die als Langloch ausgebildete Aussparung 118 einen Durchbruch auf, der insbesondere durch die erste Innenfläche 109, die zweite Innenfläche 110 und eine zylindermantelförmige Fläche 121, begrenzt ist. Dabei ist zwischen der Oberseite 103 des Mittelstücks 100 und der zylindermantelförmigen Fläche 121, der ersten Innenfläche 109 und der zweiten Innenfläche 110 ein abgerundeter Randlauf 122 ausgebildet. Durch den abgerundeten Randlauf 122 ist eine Kantenverrundung gegeben, die beim Eingreifen des Führungselements 13 in die zweite Komponente 2 mittels des Lagerkörpers 30 das Verriegeln der ersten Komponente 1 in dem Lagerkörper 30 mittels des Verriegelungskopfes 91 erleichtert. Zum Einbringen des Führungselements 13 in den Lagerkörper 30 wird zunächst die Längsachse 92 des Verriegelungskopes 91 mit der Längsachse 123 der nutförmigen Aussparung 113 zur Deckung gebracht, so daß seine Seitenflächen 97, 98 parallel zu den Innenflächen 109, 110 orientiert sind, wodurch er sich an den Absätzen 116, 117 vorbei in den Lagerkörper 30 einbringen läßt. Da in dieser Stellung die Längsachse 17 des Führungselements 13 gegenüber der Längsachse 123 der nutförmigen Aussparung 113 verschwenkt ist, wird das Einbringen dabei durch Anschlagen der Flächen 94, 95 des Führungselements 13 an der Unterseite 130 des ersten Seitenstücks 101 bzw. an der Unterseite 131 des zweiten Seitenstücks 102 begrenzt. Durch die Länge des Verbindungszapfens 96 ist allerdings der Verriegelungskopf 91 vollständig innerhalb der zylinderförmige Aussparung 114, insbesondere oberhalb der Stirnfläche 115 der zylinderförmige Aussparung 114, angeordnet. Somit kann im nächsten Schritt die Längsachse 17 des Führungselements 13 parallel zu der Länsachse 123 der nutförmigen Aussparung 113 orientiert werden, wobei der Verriegelungskopf 91 die Vorsprünge 116, 117 hintergreift. Anschließend läßt sich das Führungselement 13 zumindest teilweise in die nutförmige Aussparung 113 einbringen, wobei in der Endposition, d. h. im montierten Zustand, der Verriegelungskopf 91 über die Oberseite 103 des Mittelstücks 100 ragt. Bei einer Verschiebung des Führungselements 13 entlang der Längsachse 123 zur nutförmigen Aussparung 113 gleitet im Ausführungsbeispiel die Unterseite des Verriegelungskopfes 91 auf den Führungsflächen 119, 120 des Mittelstücks 100. Es kann auch ein Abstand zwischen den Flächen 119, 120 und der Aussparung 113 vorhanden sein. Hierfür ist es besonders vorteilhaft, daß der Randlauf 122 abgerundet ausgebildet ist, wodurch die Verschiebbarkeit, insbesondere zwischen einer Stellung, in der sich das Führungselement 13 oberhalb der zylinderförmige Aussparung 114 befindet, und einer Stellung, in der sich das Führungselement 13 zumindest teilweise oberhalb des an die zylinderförmige Aussparung 114 angrenzenden Bereichs der als Langloch ausgebildeten Aussparung 118 befindet, verbessert ist.

Durch die Anordnung der zylinderförmigen Aussparung 114 und der als Langloch ausgebildeten Aussparung 118, insbesondere auch durch die Länge der als Langloch ausgebildeten Aussparung 118, ist die translative Beweglichkeit des Führungselements 13 entlang der Längsachse 123 der nutförmigen Aussparung 113 in dem Lagerkörper 30 vorgebbar. Hierfür können an dem Mittelstück 100, insbesondere innerhalb der zylinderförmige Aussparung 114 oder innerhalb der als Langloch ausgebildeten Aussparung 118, geeignete, an den Verbindungszapfen 96 angepaßte Anschlagflächen vorgesehen sein. Der Anschlag kann ein- oder beidseitig entsprechend den z. B. in den Figuren 2B und 4C dargestellten Ausführungsbeispielen auch an einer Anschlagfläche 23 (Fig. 2B) der zweiten Komponente 2 begrenzt sein. Außerdem kann die nutförmige Aussparung 113 entlang der Längsachse 123 ein- oder beidseitig innerhalb des Lagerkörpers 30 begrenzt sein.

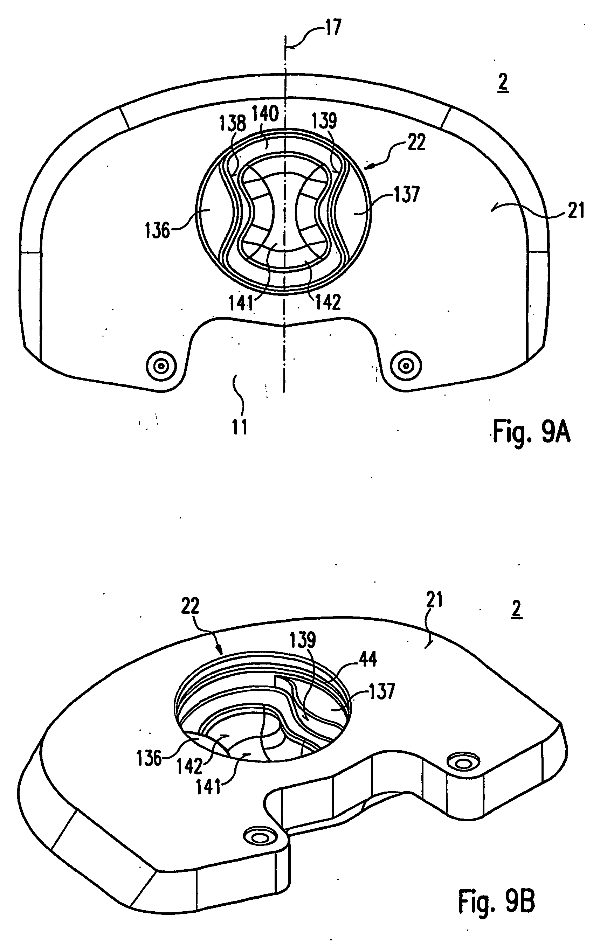

Fig. 9A zeigt die zweite Komponente 2 der Gelenkprothese gemäß dem fünften Ausführungsbeispiel der Erfindung, in die der in den Fig. 8A und 8B dargestellte Lagerkörper 30 einsetzbar ist. Fig. 9B zeigt die in Fig. 9A dargestellte zweite Komponente in einer räumlichen Perspektive.

Die zweite Komponente 2 weist die Aussparung 22 auf, in die der Lagerkörper 30 vollständig einbringbar ist. Dadurch kann die Unterseite 130 des ersten Seitenstücks 101 und die Unterseite 131 des zweiten Seitenstücks 102 des Lagerkörpers 30 unterhalb der Ebenen angeordnet werden, in der die Lagerfläche 21 der zweiten Komponente 2 liegt, so daß die zweite Komponente 2 im montierten Zustand der Gelenkprothese auf der Lagerplatte 5 der ersten Komponente 1 aufliegt. Die Aussparung 22 weist eine umlaufende Nut 44 auf, in die der umlaufende Kreisbund 41 des Lagerkörpers 30 im montierten Zustand der Gelenkprothese eingreift, wobei das Eingreifen analog zu dem in Fig. 3 dargestellten ersten Ausführungsbeispiel erfolgt.

Die zweite Komponente 2 weist bezüglich der Längsachse 17 symmetrisch angeordnete Längskörper 136, 137 auf, die im Bereich der Aussparung 22 vorgesehen sind. Im montierten Zustand der Gelenkprothese ist die Längsseite 106 des Mittelstück 100 des Lagerkörpers 30 bezüglich der Längsachse 17 zumindest im Wesentlichen auf der Seite des Längskörpers 136 angeordnet, während die Längsseite 107 des Mittelstücks 100 des Lagerkörpers 30 zumindest im Wesentlichen auf der Seite des Längskörpers 137 angeordnet ist. An den Längskörpern 136, 137 sind Längsflächen 138, 139 ausgebildet, die zumindest im Wesentlichen gegenüberliegend zu den Längsseiten 106, 107 liegen, wobei die Drehung des montierten Lagerkörpers 30 in der Aussparung 22 durch Anschlagen der Längsseite 106 des Mittelstücks 100 des Lagerkörpers 30 an der Längsfläche 138 des Längskörpers 136 und der Längsseite 107 des Mittelstücks 100 des Lagerkörpers 30 an der Längsfläche 139 des Längskörpers 137 begrenzt ist.

Um eine Anpassung an den anatomischen Bereich der Drehbeweglichkeit eines natürlichen Gelenks, insbesondere eines Kniegelenks, zu erreichen, sind die Längskörper 136, 137 bauchförmig ausgebildet.

Ferner ist im Bereich der Aussparung 22 eine ringförmige Fläche 140 vorgesehen, die parallel zu der Lagerfläche 21 der zweiten Komponente ist. Im montierten Zustand der Gelenkprothese ist die ringförmige Fläche 140 zumindest teilweise gegenüberliegend zu der Oberseite 103 des Mittelstücks 100 des Lagerkörpers 30 angeordnet. Der Lagerkörper 30 kann sich ggf. an der Oberseite 104 des ersten Seitenstücks 101 und an der Oberseite 105 des zweiten Seitenstücks 102 an den Längskörpern 136, 137 und/oder über die Oberseite 103 des Mittelstücks 100 an der ringförmigen Fläche 140 der zweiten Komponente 2 abstützen.

Die größte Tiefe weist die Aussparung 22 an einer Bodenfläche 141 auf. Zwischen der Bodenfläche 141 und der ringförmigen Fläche 140 ist eine Stufe ausgebildet, wobei der Übergang abgerundet ausgeführt ist, so daß eine mehrfach gekrümmte Flache 142 ausgebildet ist. Die Krümmung der Fläche 142 und die Oberfläche 143 des in den Fig. 7A und 7B dargestellten Verriegelungskopfes 91 sind aufeinander abgestimmt. Durch Anschlagen des Verriegelungskopfes 91 an der Fläche 142 kann die Bewegung des Verriegelungskopfes 91 zum Begrenzen der Translationsbewegung des Führungselements 13 entlang der Längsachse 17 in Abhängigkeit von der Drehwinkelstellung der ersten Komponente 1 bezüglich der zweiten Komponente 2 eingeschränkt werden.

Es bestehen daher mehrere Möglichkeiten, die translative Bewegung des Führungselements 13 zu begrenzen, wobei in Abhängigkeit von der Drehwinkelstellung der ersten Komponente 1 bezüglich der zweiten Komponente 2 die Begrenzung auch jeweils unterschiedlich erreicht werden kann. Bezüglich der Möglichkeiten der Begrenzung der translativen Bewegung kann auf die bereits behandelten Möglichkeiten verwiesen werden, die auch miteinander kombiniert werden können.

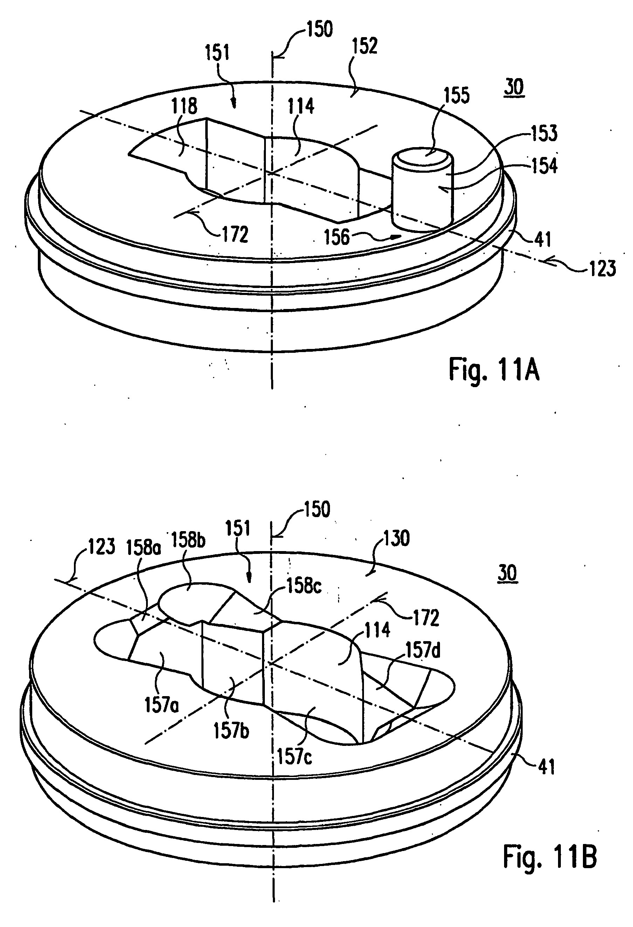

Fig. 10 zeigt die Gelenkprothese gemäß dem fünften Ausführungsbeispiel im montierten Zustand, wobei die zweite Komponente 2 in einer geschnittenen Ansicht dargestellt ist.

Die in Fig. 10 dargestellte zweite Komponente 2 der Gelenkprothese weist an ihrer Oberseite 3 eine Lagerschale 144 auf, in der sich der zweite Knochen, z. B. mittels einer geeigneten Kondylenprothese, abstützt.

Durch den Lagerkörper 30 ist eine Drehung der zweiten Komponente 2 relativ zu der ersten Komponente 1 ermöglicht. Außerdem kann die zweite Komponente 2 relativ zu der ersten Komponente 1 translativ bewegt werden, wobei die Bewegung durch Anschlagen der Oberfläche 143 des Verriegelungskopfes 91 an der mehrfach gekrümmten Fläche 142 der zweiten Komponente 2 begrenzt ist.

Die Figuren 11A, 11B zeigen einen Lagerkörper 30 gemäß einem sechsten Ausführungsbeispiel der Erfindung. Der Lagerkörper 30 gemäß dem sechsten Ausführungsbeispiel ist insbesondere als Alternative zu dem Lagerkörper gemäß dem fünften Ausführungsbeispiel, der in den Fig. 8A und 8B dargestellt ist, gedacht. Speziell eignet sich der Lagerkörper 30 gemäß dem sechsten Ausführungsbeispiel in Zusammenwirkung mit der ersten Komponente 1 der Gelenkprothese gemäß dem fünften Ausführungsbeispiel, die in den Figuren 7A und 7B dargestellt ist.

Der Lagerkörper 30 weist eine Drehachse 150 auf, um die der Lagerkörper 30 im montierten zustand der Gelenkprothese drehbar ist. Der Lagerkörper 30 umfaßt eine Ausparung 151, die im Wesentliche symmetrisch zur Drehachse 150 ausgebildet ist. Die Aussparung 151 umfaßt eine zylinderförmige Aussparung 114, die bezüglich der Drehachse 150 des Lagerkörpers 30 symmetrisch angeordnet ist. Ferner umfaßt die Aussparung 151 eine als Langloch ausgebildeten Aussparung 118, die symmetrisch zu deren Längsachse 123 ausgebildet ist.

Auf der Oberseite 152 des Lagerkörpers 30 ist ein auf der Längsachse 123 der als Langloch ausgebildeten Aussparung 118 angeordneter zylinderförmiger Stift 153 befestigt, der eine Zylindermantelfläche 154 und eine Stirnfläche 155 umfaßt. Der Stift 153 ist daher im Randbereich 156 der Oberseite 152 des Lagerkörpers 30 angeordnet.

Um das Einbringen des Führungselements 13 und des mit diesem verbundenen Verriegelungskopfes 91 in die Aussparung 151 zum Montieren der Gelenkprothese zu vereinfachen, sind mehrere Kantenabschrägungen, die auch als Kantenabrundungen ausgebildet sein können, zwischen der Unterseite 130 des Lagerkörpers 30 und den Innenflächen (z. B. 157a bis 157d) der Aussparung 151, die senkrecht zur Unterseite 130 orientiert sind, vorgesehen. Beispiele für diese Abschrägungen sind mit 158a bis 158c bezeichnet.

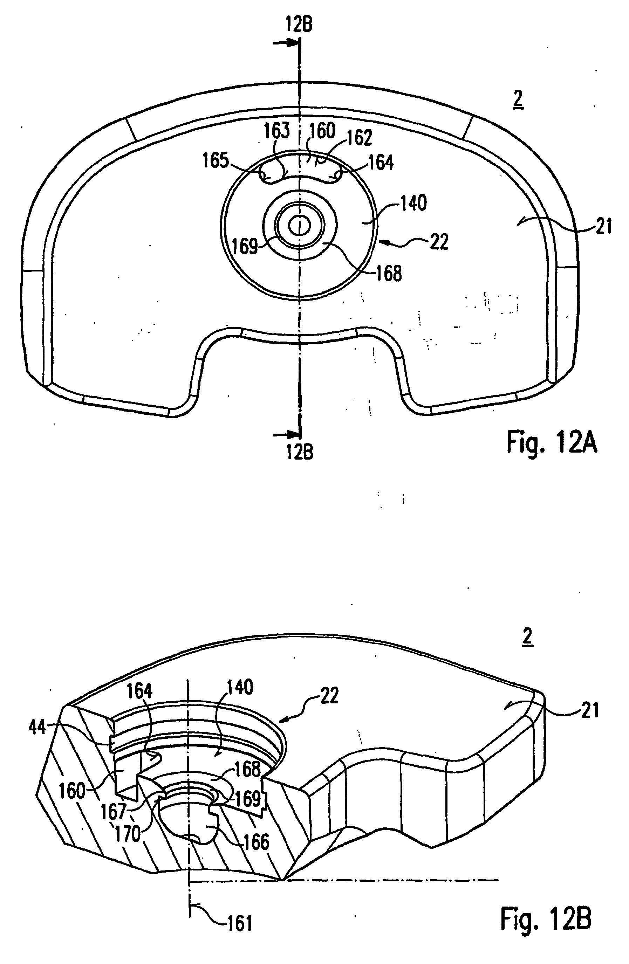

Die Figuren 12A und 12B zeigen eine zweite Komponente 2 gemäß einem sechsten Ausführungsbeispiel der Erfindung. Die zweite Komponente 2 gemäß dem sechsten Ausführungsbeispiel der Erfindung ist insbesondere als Alternative zu der zweiten Komponente 2 gemäß dem fünften Ausführungsbeispiel der Erfindung, die in den Fig. 9A und 9B dargestellt ist, in Zusammenwirkung mit dem Lagerkörper 30 gemäß dem sechsten Ausführungsbeispiel, der in den Fig. 11A und 11B dargestellt ist, gedacht. Speziell eignet sich die zweite Komponente 2 gemäß dem sechsten Ausführungsbeispiel in Zusammenwirkung mit dem Lagerkörper 30 gemäß dem sechsten Ausführungsbeispiel, der in den Fig. 11A und 11B dargestellt ist, für die Montage mit der ersten Komponente 1 gemäß dem fünften Ausführungsbeispiel, die in den Fig. 7A und 7B dargestellt ist, zu einer erfindungsgemäßen Gelenkprothese.

Die Fig. 12B zeigt dabei eine perspektivische Schnittdarstellung entlang der in Fig. 12A mit 12B bezeichneten Schnittlinie.

Die zweite Komponente 2 weist eine Aussparung 22 auf, die ein gebogenes Langloch 160 umfaßt. Das gebogen Langloch 160 ist dabei symmetrisch bezüglich der Mittelachse 161 der Aussparung 22 gebogen. Das Langloch 160 weist eine äußere Führungsfläche 162 und eine innere Führungsfläche 163 auf. Die Führungsflächen 162, 163 sind an den Enden des Langlochs 160 mittels zumindest im wesentlichen zylindermantelförmig gebogenen Anschlagflächen 164, 165, die an die Zylindermantelfläche 154 des Stiftes 153 angepaßt sind, miteinander verbunden.

Im montierten Zustand der Gelenkprothese greift der zylinderische Stift 153 in das Langloch 160 ein, so daß die Drehbeweglichkeit des Lagerkörpers 30 durch Anschlagen des Stiftes 153 mittels der Zylindermantelfläche 154 an den Anschlagflächen 164, 165 des gebogenen Langloches 160 begrenzt ist.

Die zweite Komponente 2 weist außerdem einen Sackraum 166 auf, der an die Aussparung 22 im Bereich einer durch einen umlaufenden Bund 167 gegebenen Einschnürung angrenzt. Der Sackraum 166 dient zum Einbringen des Verriegelungskopfes 91, wobei der Verriegelungskopf 91 durch die durch den Bund 167 gegebene Einschnürung gepreßt wird und anschließend einschnappt. Dadurch wird eine Verriegelung zwischen der ersten Komponente 1 und der zweiten Komponente 2 geschaffen.

Im montierten Zustand der Gelenkprothese kann sich der Lagerkörper 30 mittels seiner Oberseite 152 ggf. an der ringförmigen Fläche 140 abstützen.

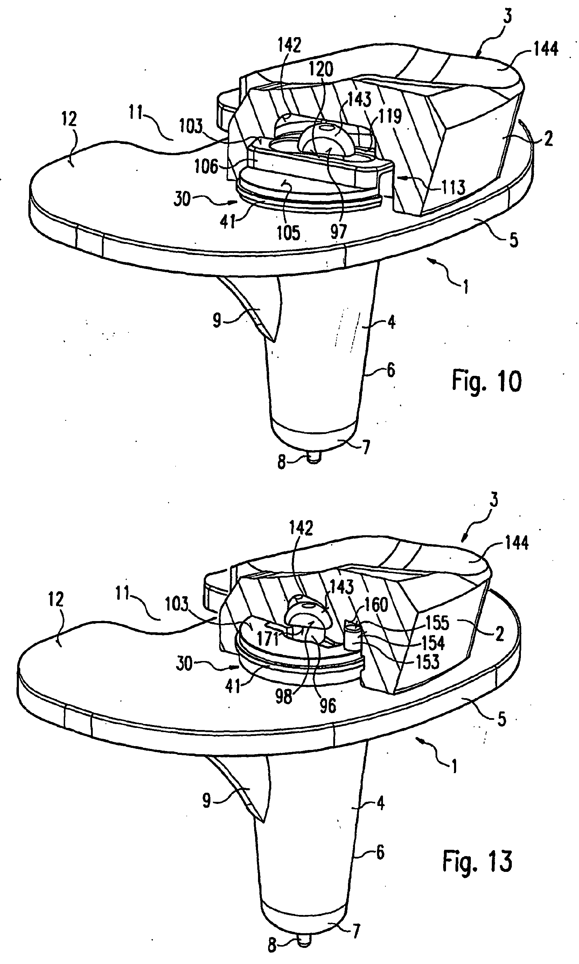

Um das Einbringen des Verriegelungskopfes 91 in den Sackraum 166 zu erleichtern, ist der Bund 167 mittels Fasen 168, 169 abgeschrägt und der Verriegelungskopft 91 an seiner Oberfläche 143 rund ausgebildet. Im eingeschnappten Zustand wirkt die zumindest im Randbereich des Verriegelungskopfes 91 eben ausgebildete Unterseite 171 (Fig. 13) mit der parallel zu der Mittelachse 161 orientierten Anschlagfläche 170 des Sackraumes 166 zusammen, so daß eine unlösbare Verbindung zwischen der ersten Komponente 1 und der zweiten Komponente 2 geschaffen ist.

Die in den Figuren 11A und 11B dargestellte Aussparung 151, die insbesondere durch die Flächen 157a bis 157d gegeben ist, ist an die Form des Führungselementes 13 angepaßt. Speziell sind die Innenfläche 157a und die dieser bezüglich der Achse 172 gegenüberliegende Innenfläche an die Stirnflächen 62, 63 des Führungselementes 13 angepaßt. Ferner sind die Innenfläche 157b, 157d und die diesen bezüglich der Längsachse 123 gegenüberliegenden Innenflächen an die Seitenfläche 66, 67 des Führungselementes 13 angepaßt. Dadurch ist eine Passung des Führungselements 13 in die Aussparung 151 gegeben. Die Passung kann insbesondere formschlüssig ausgeführt sein.

Fig. 13 zeigt die Gelenkprothese im montierten Zustand gemäß dem sechsten Ausführungsbeispiel. Die montierte Gelenkprothese gemäß dem sechsten Ausführungsbeispiel besteht aus der ersten Komponente 1 gemäß dem fünften Ausführungsbeispiel, die in den Fig. 7A und 7B dargestellt ist, dem Lagerkörper 30 gemäß dem sechsten Ausführungsbeispiel, der in den Fig. 11A und 11B dargestellt ist, und der zweiten Komponente 2 gemäß dem sechsten Ausführungsbeispiel, die in den Fig. 12A und 12B dargestellt ist. In Fig. 13 ist dabei die zweite Komponente 2 entsprechend der Darstellung in Fig. 12B als eine entlang der in Fig. 12A mit 12B bezeichneten Schnittlinie geschnittene Ansicht dargestellt.

Die Erfindung ist nicht auf die beschriebenen Ausführungsbeispiele beschränkt. Insbesondere können für die Bauteile der Gelenkprothese verschiedenste Materialien zum Einsatz kommen. Insbesondere kann der Lagerkörper 30 aus einem Metall, insbesondere Kobalt-Chrom-Legierung, Titan, Keramik oder einem Polymer, oder auch aus mehreren Materialien bestehen.

## Patentansprüche

1. Gelenkprothese, insbesondere Kniegelenkprothese, zum Ausbilden einer Gelenkverbindung zwischen einem ersten Knochen und zumindest einem zweiten Knochen mit
einer ersten Komponente (1), die zumindest mittelbar mit dem ersten Knochen verbunden ist,
zumindest einer zweiten Komponente (2), auf der sich der zweite Knochen zumindest mittelbar abstützt und die auf zumindest einer Lagerfläche (12) der ersten Komponente (1) bewegbar gelagert ist, und
einem Führungselement (13), das mit der ersten Komponente (1) verbunden ist und in die zweite Komponente (2) eingreift, um eine translative Bewegung der zweiten Komponente (2) relativ zu der ersten Komponente (1) zu begrenzen,
**dadurch gekennzeichnet,**
**daß** das Führungselement (13) mittels eines in der zweiten Komponente (2) drehbar gelagerten Lagerkörpers (30) in die zweite Komponente (2) eingreift, wobei der Lagerkörper (30) eine Drehung des Führungselementes (13) relativ zu der zweiten Komponente (2) ermöglicht.

2. Gelenkprothese nach Anspruch 1,
**dadurch gekennzeichnet,**
**daß** der Lagerkörper (30) eine zylinderförmige Grundform aufweist.

3. Gelenkprothese nach Anspruch 2,
**dadurch gekennzeichnet,**
**daß** der Lagerkörper (30) in einer zumindest im wesentlichen zylinderförmig ausgebildeten Aussparung (22) der zweiten Komponente (2) gelagert ist.

4. Gelenkprothese nach Anspruch 3,
**dadurch gekennzeichnet,**
**daß** der Lagerkörper (30) zumindest einen seitlichen Bund (41) aufweist, der in eine mit der zylinderförmigen Aussparung (22) verbundene Nut der zweiten Komponente (2) eingreift.

5. Gelenkprothese nach Anspruch 4,
**dadurch gekennzeichnet,**
**daß** der seitliche Bund (41) des Lagerkörpers (30) als umlaufender Kreisbund ausgebildet ist.

6. Gelenkprothese nach Anspruch 4,
**dadurch gekennzeichnet,**
**daß** der seitliche Bund (41) des Lagerkörpers (30) als abschnittsweise umlaufender Kreisbund ausgebildet ist.

7. Gelenkprothese nach Anspruch 6,
**dadurch gekennzeichnet,**
**daß** die in der zweiten Komponente (2) ausgebildete Nut (44) umfänglich abschnittsweise ausgebildet ist, um die Drehbewegung des Führungselementes (13) relativ zu der zweiten Komponente (2) zu begrenzen.

8. Gelenkprothese nach Anspruch 5 oder 6,
**dadurch gekennzeichnet,**
**daß** die in der zweiten Komponente (2) ausgebildete Nut (44) umfänglich umlaufend ausgebildet ist, um eine unbegrenzte Drehbeweglichkeit des Führungselementes (13) relativ zu der zweiten Komponente (2) zu gewährleisten.

9. Gelenkprothese nach einem der Ansprüche 1 bis 8,
**dadurch gekennzeichnet,**
**daß** der Lagerkörper (30) zumindest eine erste Aussparung (31) aufweist, in der das Führungselement (13) geführt ist.

10. Gelenkprothese nach Anspruch 9,
**dadurch gekennzeichnet,**
**daß** die erste Aussparung (31) als eine auf der Seite der Lagerfläche (12) der ersten Komponente (1) angeordnete erste Nut ausgebildet ist.

11. Gelenkprothese nach Anspruch 9 oder 10,
**dadurch gekennzeichnet,**
**daß** das Führungselement (13) in der ersten Aussparung (31) in einer Längsrichtung (17, 48) der ersten Aussparung (31) bewegbar ist, um eine translative Bewegung des Führungselementes (13) zu ermöglichen.

12. Gelenkprothese nach Anspruch 11,
**dadurch gekennzeichnet,**
**daß** die erste Aussparung (31) zumindest in einer Längsrichtung (17, 48) eine Anschlagfläche (60, 61) aufweist, die die translative Bewegung des Führungselementes (13) in der ersten Aussparung (31) begrenzt.

13. Gelenkprothese nach-Anspruch 12,
**dadurch gekennzeichnet,**
**daß** der Lagerkörper (30) zumindest eine seitlich der Anschlagfläche (60, 61) der ersten Aussparung (31) angeordnete Kantenausformung (64a, 64b, 64e, 64f) aufweist, die mit der ersten Aussparung (31) verbunden ist.

14. Gelenkprothese nach Anspruch 13,
**dadurch gekennzeichnet,**
**daß** die Kantenausformung (64a, 64b, 64e, 64f) als Bohrung ausgebildet ist.

15. Gelenkprothese nach einem der Ansprüche 1 bis 14,
**dadurch gekennzeichnet,**
**daß** der Lagerkörper (30) eine zweite Aussparung (32) aufweist, in die das Führungselement (13) einsetzbar ist, wobei der Lagerkörper (30) in der zweiten Aussparung (32) zumindest im wesentlichen fixiert ist.

16. Gelenkprothese nach Anspruch 15,
**dadurch gekennzeichnet,**
**daß** die zweite Aussparung (32) als eine auf der Seite der Lagerfläche (21) der ersten Komponente (1) angeordnete Nut ausgebildet ist.

17. Gelenkprothese nach Anspruch 15 oder 16,
**dadurch gekennzeichnet,**
**daß** der Lagerkörper (30) zumindest eine in einer Kante der zweiten Aussparung (32) angeordnete Kantenausformung (64c, 64d, 64g, 64h) aufweist, die mit der zweiten Aussparung verbunden ist.

18. Gelenkprothese nach Anspruch 17,
**dadurch gekennzeichnet,**
**daß** die Kantenausformung (64c, 64d, 64g, 64h) als Bohrung ausgebildet ist.

19. Gelenkprothese nach einem der Ansprüche 1 bis 18,
**dadurch gekennzeichnet,**
**daß** das Führungselement (13) einen im wesentlichen rechteckigen Querschnitt aufweist.

20. Gelenkprothese nach einem der Ansprüche 1 bis 19,
**dadurch gekennzeichnet,**
**daß** das Führungselement (13) einen Querschnitt mit abgerundeten Kanten (71a - 71d) aufweist.

21. Gelenkprothese nach Anspruch 20,
**dadurch gekennzeichnet,**
**daß** das Führungselement (13) einen Querschnitt mit zumindest einem abgerundeten Ende (72, 73) aufweist.

22. Gelenkprothese nach einem der Ansprüche 1 bis 6,
**dadurch gekennzeichnet,**
**daß** das Führungselement (13) mit einem Verriegelungskopf (91) verbunden ist, der in den Lagerkörper (30) eingreift, um die erste Komponente (1) in dem Lagerkörper (30) zu verriegeln.

23. Gelenkprothese nach Anspruch 22,
**dadurch gekennzeichnet,**
**daß** die Längsachse (92) des Verriegelungskopfes (91) bezüglich der Längsachse (17) des Führungselementes (13) um einen Schwenkwinkel (α) verschwenkt ist.

24. Gelenkprothese nach Anspruch 22 oder 23,
**dadurch gekennzeichnet,**
**daß** der Lagerkörper (30) zumindest eine Führungsfläche (119, 120) aufweist, an der der Verriegelungskopf (91) bei der translativen Bewegung der zweiten Komponente (2) relativ zu der ersten Komponente (1) geführt ist.

25. Gelenkprothese nach einem der Ansprüche 22 bis 24,
**dadurch gekennzeichnet,**
**daß** die zweite Komponente zumindest einen Längskörper (136, 137) aufweist, der zumindest eine Längsfläche (138, 139) umfaßt, und
**daß** die Drehung des Führungselementes (13) relativ zu der zweiten Komponente (2) durch Anschlagen zumindest einer Längsseite (106, 107) des Lagerkörpers (30) an der Längsfläche (138, 139) begrenzt ist.

26. Gelenkprothese nach Anspruch 25,
**dadurch gekennzeichnet,**
**daß** die Längsseite (106, 107) an einem Mittelstück (100) des Lagerkörpers (30) ausgebildet ist.

27. Gelenkprothese nach einem der Ansprüche 22 bis 26,
**dadurch gekennzeichnet,**
**daß** die zweite Komponente (2) eine mehrfach gekrümmte Fläche (142) aufweist, die bei einer translativen Bewegung der zweiten Komponente (2) relativ zu der ersten Komponente (1) und/oder einer Drehung des Führungselementes (13) relativ zu der zweiten Komponente (2) die Bewegung des Verriegelungskopfes (91) begrenzt zum Begrenzen der Translativbewegung oder der Drehbewegung.

28. Gelenkprothese nach einem der Ansprüche 1 bis 6,
**dadurch gekennzeichnet,**
**daß** das Führungselement (13) mit einem Verriegelungskopf (91) verbunden ist, der zum Verbinden der ersten Komponente (1) mit der zweiten Komponente (2) in die zweite Komponente (2) eingreift.

29. Gelenkprothese nach Anspruch 28,
**dadurch gekennzeichnet,**
**daß** der Verriegelungskopf (91) in einen Sackraum (166) der zweiten Komponente (2) eingreift, wobei eine Entfernung des Verriegelungskopfes (91) durch Anschlagen des Verriegelungskopfes (91) an einer Anschlagfläche (170) des Sackraums (166) verhindert ist.

30. Gelenkprothese nach Anspruch 28 oder 29,
**dadurch gekennzeichnet,**
**daß** die zweite Komponente (2) ein Langloch (160) aufweist,
**daß** der Lagerkörper (30) mit einem Stift (153) verbunden ist, der in das Langloch (160) eingreift, und
**daß** die Drehung des Führungselementes (13) relativ zu der zweiten Komponente (2) durch Anschlagen der Zylindermantelfläche (154) des Stiftes (153) an den Anschlagflächen (164, 165) des Langlochs (160) begrenzt ist. -

31. Gelenkprothese nach Anspruch 30,
**dadurch gekennzeichnet,**
**daß** das Langloch (160) bezüglich der Mittelachse (161) der zweiten Komponente (2) radial gekrümmt ist, wobei die Mittelachse (161) der zweiten Komponente (2) im montierten Zustand der Gelenkprothese zumindest im Wesentlichen mit der Drehachse (150) des Lagerkörpers (30) übereinstimmt.

32. Gelenkprothese nach einem der Ansprüche 22 bis 31,
**dadurch gekennzeichnet,**
**daß** die Oberfläche (143) des Verriegelungskopfes (91) gekrümmt ausgebildet ist, wobei die Krümmung der Oberfläche (143) an die Krümmung einer mehrfach gekrümmten Fläche (142) der zweiten Komponente (2), die den Verriegelungskopf (91) zumindest im Wesentlichen umgibt, angepaßt ist.

33. Gelenkprothese nach einem der Ansprüche 22 bis 32,
**dadurch gekennzeichnet,**
**daß** der Verriegelungskopf (91) zumindest eine Seitenfläche (97, 98) aufweist, die in einer vorgegebenen Drehstellung des Verriegelungskopfes (91) mit einer Innenfläche (109, 110) des Lagerkörpers (30) fluchtet zum Lösen des Eingriffs des Verriegelungskopfes (91) in den Lagerkörper (30).

## Claims

1. Joint prosthesis, in particular knee joint prosthesis, for producing a joint connection between a first bone and at least one second bone, comprising
a first component (1) which is at least indirectly connected to the first bone,
at least one second component (2), on which the second bone is at least indirectly supported and which is movably mounted on at least one bearing surface (12) of the first component (1), and
a guide element (13) which is connected to the first component (1) and engages in the second component (2), in order to limit a translational movement of the second component (2) relative to the first component (1),
**characterised in that**
the guide element (13) engages in the second component (2) by means of a bearing body (30) rotatably mounted in the second component (2), the bearing body (30) allowing a rotation of the guide element (13) relative to the second component (2).

2. Joint prosthesis according to Claim 1, **characterised in that** the bearing body (30) has a cylindrical basic shape.

3. Joint prosthesis according to Claim 2, **characterised in that** the bearing body (30) is mounted in an at least substantially cylindrically configured recess (22) of the second component (2).

4. Joint prosthesis according to Claim 3, **characterised in that** the bearing body (30) comprises at least one lateral collar (41) which engages in a groove of the second component (2) joined to the cylindrical recess (22).

5. Joint prosthesis according to Claim 4, **characterised in that** the lateral collar (41) of the bearing body (30) is configured as a peripheral circular collar.

6. Joint prosthesis according to Claim 4, **characterised in that** the lateral collar (41) of the bearing body (30) is configured as a partially peripheral circular collar.

7. Joint prosthesis according to Claim 6, **characterised in that** the groove (44) configured in the second component (2) is configured to be partially peripheral, in order to limit the rotational movement of the guide element (13) relative to the second component (2).

8. Joint prosthesis according to Claim 5 or 6, **characterised in that** the groove (44) configured in the second component (2) is configured to be substantially peripheral, in order to ensure an unlimited rotational mobility of the guide element (13) relative to the second component (2).

9. Joint prosthesis according to one of Claims 1 to 8, **characterised in that** the bearing body (30) comprises at least one first recess (31), in which the guide element (13) is guided.

10. Joint prosthesis according to Claim 9, **characterised in that** the first recess (31) is configured as a first groove arranged on the side of the bearing surface (12) of the first component (1).

11. Joint prosthesis according to Claim 9 or 10, **characterised in that** the guide element (13) is movable in the first recess (31) in a longitudinal direction (17, 48) of the first recess (31), in order to allow a translational movement of the guide element (13).

12. Joint prosthesis according to Claim 11, **characterised in that** the first recess (31) comprises, at least in one longitudinal direction (17, 48), a stop surface (60, 61), which limits the translational movement of the guide element (13) in the first recess (31).

13. Joint prosthesis according to Claim 12, **characterised in that** the bearing body (30) comprises at least one edge recess (64a, 64b, 64e, 64f) arranged to the side of the stop surface (60, 61) of the first recess (31), and which is connected to the first recess (31).

14. Joint prosthesis according to Claim 13, **characterised in that** the edge recess (64a, 64b, 64e, 64f) is configured as a bore.

15. Joint prosthesis according to one of Claims 1 to 14, **characterised in that** the bearing body (30) comprises a second recess (32), into which the guide element (13) may be inserted, the bearing body (30) being at least substantially fixed in the second recess (32).

16. Joint prosthesis according to Claim 15, **characterised in that** the second recess (32) is configured as a groove arranged on the side of the bearing surface (21) of the first component (1).

17. Joint prosthesis according to Claim 15 or 16, **characterised in that** the bearing body (30) comprises at least one edge recess (64c, 64d, 64g, 64h) arranged in one edge of the second recess (32), which is connected to the second recess.

18. Joint prosthesis according to Claim 17, **characterised in that** the edge recess (64c, 64d, 64g, 64h) is configured as a bore.

19. Joint prosthesis according to one of Claims 1 to 18, **characterised in that** the guide element (13) has a substantially rectangular cross-section.

20. Joint prosthesis according to one of Claims 1 to 19, **characterised in that** the guide element (13) has a cross-section with rounded edges (71a - 71d).

21. Joint prosthesis according to Claim 20, **characterised in that** the guide element (13) has a cross-section with at least one rounded end (72, 73).

22. Joint prosthesis according to one of Claims 1 to 6, **characterised in that** the guide element (13) is connected to a locking head (91) which engages in the bearing body (30), in order to lock the first component (1) in the bearing body (30).

23. Joint prosthesis according to Claim 22, **characterised in that** the longitudinal axis (92) of the locking head (91) is pivoted relative to the longitudinal axis (17) of the guide element (13) about a pivot angle (α).

24. Joint prosthesis according to Claim 22 or 23, **characterised in that** the bearing body (30) comprises at least one guide surface (119, 120) on which the locking head (91) is guided relative to the first component (1) during the translational movement of the second component (2).

25. Joint prosthesis according to one of Claims 22 to 24, **characterised in that** the second component comprises at least one longitudinal body (136, 137) which comprises at least one longitudinal surface (138, 139), and **in that** the rotation of the guide element (13) relative to the second component (2) is limited by at least one longitudinal side (106, 107) of the bearing body (30) bearing against the longitudinal surface (138, 139).

26. Joint prosthesis according to Claim 25, **characterised in that** the longitudinal side (106, 107) is configured on a central part (100) of the bearing body (30).

27. Joint prosthesis according to one of Claims 22 to 26, **characterised in that** the second component (2) comprises a repeatedly curved surface (142), which limits the movement of the locking head (91) during a translational movement of the second component (2) relative to the first component (1) and/or a rotation of the guide element (13) relative to the second component (2), for limiting the translational movement or the rotational movement.

28. Joint prosthesis according to one of Claims 1 to 6, **characterised in that** the guide element (13) is connected to a locking head (91) which engages in the second component (2) for connecting the first component (1) to the second component (2).

29. Joint prosthesis according to Claim 28, **characterised in that** the locking head (91) engages in a blind space (166) of the second component (2), a removal of the locking head (91) being prevented by the locking head (91) bearing against a stop surface (170) of the blind space (166).

30. Joint prosthesis according to Claim 28 or 29, **characterised in that** the second component (2) comprises a slot (160), **in that** the bearing body (30) is connected to a pin (153) which engages in the slot (160) and **in that** the rotation of the guide element (13) relative to the second component (2) is limited by the cylindrical outer surface (154) of the pin (153) bearing against the stop surfaces (164, 165) of the slot (160).

31. Joint prosthesis according to Claim 30, **characterised in that** the slot (160) is radially curved relative to the centre axis (161) of the second component (2), the centre axis (161) of the second component (2) in the assembled state of the joint prosthesis at least substantially coinciding with the rotational axis (150) of the bearing body (30).

32. Joint prosthesis according to one of Claims 22 to 31, **characterised in that** the surface (143) of the locking head (91) is of curved configuration, the curvature of the surface (143) being adapted to the curvature of a repeatedly curved surface (142) of the second component (2) which at least substantially surrounds the locking head (91).

33. Joint prosthesis according to one of Claims 22 to 32, **characterised in that** the locking head (91) has at least one lateral surface (97, 98) which, in a predetermined rotary position of the locking head (91), is aligned with an internal surface (109, 110) of the bearing body (30) for releasing the engagement of the locking head (91) in the bearing body (30).

## Revendications

1. Prothèse articulaire, en particulier prothèse articulaire du genou, pour réaliser un joint articulaire entre un premier os et au moins un deuxième os avec
un premier composant (1) qui est relié au moins indirectement au premier os,
au moins un deuxième composant (2) sur lequel le deuxième os s'appuie au moins indirectement et qui est logée de manière mobile sur au moins une surface d'appui (12) du premier composant (1) et
un élément de guidage (13) qui est relié au premier composant (1) et entre en prise dans le deuxième composant (2), afin de limiter un mouvement de translation du deuxième composant (2) par rapport au premier composant (1),
**caractérisée en ce que**
l'élément de guidage (13) entre en prise dans le deuxième composant (2) au moyen d'un corps d'appui (30) logé dans le deuxième composant (2) de manière à pouvoir tourner, le corps d'appui (30) permettant une rotation de l'élément de guidage (13) par rapport au deuxième composant (2).

2. Prothèse articulaire selon la revendication 1,
**caractérisée en ce que**
le corps d'appui (30) a une forme de base cylindrique.

3. Prothèse articulaire selon la revendication 2,
**caractérisée en ce que**
le corps d'appui (30) est logé dans un évidement (22) du deuxième composant (2) réalisé pour l'essentiel en forme de cylindre.

4. Prothèse articulaire selon la revendication 3,
**caractérisée en ce que**
le corps d'appui (30) comporte au moins un collet latéral (41) qui entre en prise dans une rainure du deuxième composant (2) reliée à l'évidement en forme de cylindre (22).

5. Prothèse articulaire selon la revendication 4,
**caractérisée en ce que**
le collet latéral (41) du corps d'appui (30) est réalisé sous forme d'épaulement circulaire rotatif.

6. Prothèse articulaire selon la revendication 4,
**caractérisée en ce que**
le collet latéral (41) du corps d'appui (30) est réalisé sous forme d'épaulement circulaire rotatif sur certaines sections.

7. Prothèse articulaire selon la revendication 6,
**caractérisée en ce que** la rainure (44) réalisée dans le deuxième composant (2) est réalisée sur certaines sections de son pourtour, afin de limiter le mouvement de rotation de l'élément de guidage (13) par rapport au deuxième composant (2).

8. Prothèse articulaire selon la revendication 5 ou 6,
**caractérisée en ce que** la rainure (44) réalisée dans le deuxième composant (2) est réalisée rotative sur son pourtour, afin de garantir une mobilité en rotation de l'élément de guidage (13) par rapport au deuxième composant (2).

9. Prothèse articulaire selon une des revendications 1 à 8,
**caractérisée en ce que**
le corps d'appui (30) comporte au moins un premier évidement (31) dans lequel l'élément de guidage (13) est guidé.

10. Prothèse articulaire selon la revendication 9,
**caractérisée en ce que** le premier évidement (31) est réalisé sous la forme d'une première rainure disposée du côté de la surface d'appui (12) du premier composant (1).

11. Prothèse articulaire selon la revendication 9 ou 10,
**caractérisée en ce que**
l'élément de guidage (13) dans le premier évidement (31) est mobile dans un sens longitudinal (17, 48) du premier évidement (31), afin de permettre un mouvement de translation de l'élément de guidage (13).

12. Prothèse articulaire selon la revendication 11,
**caractérisée en ce que**
le premier évidement (31) comporte au moins dans un sens longitudinal (17, 48) une surface de butée (60, 61) qui limite le mouvement de translation de l'élément de guidage (13) dans le premier évidement (31).

13. Prothèse articulaire selon la revendication 12,
**caractérisée en ce que**
le corps d'appui (30) comporte au moins un bord formé (64a, 64b, 64e, 64f) disposé latéralement par rapport à la surface de butée (60, 61) du premier évidement (31) et relie au premier évidement (31).

14. Prothèse articulaire selon la revendication 13,
**caractérisée en ce que**
le bord formé (64a, 64b, 64e, 64f) est réalisé sous forme de perçage.

15. Prothèse articulaire selon une des revendications 1 à 14,
**caractérisée en ce que**
le corps d'appui (30) comporte un deuxième évidement (32) dans lequel l'élément de guidage (13) peut être inséré, le corps d'appui (30) étant au moins pour l'essentiel fixé dans le deuxième évidement (32).

16. Prothèse articulaire selon la revendication 15,
**caractérisée en ce que**
le deuxième évidement (32) est réalisé sous forme d'une rainure disposée sur le côté de la surface d'appui (21) du premier composant (1).

17. Prothèse articulaire selon la revendication 15 ou 16,
**caractérisée en ce que**
le corps d'appui (30) comporte au moins un bord formé (64c, 64d, 64g, 64h) disposé dans un bord du deuxième évidement (32), qui est relié au deuxième évidement.

18. Prothèse articulaire selon la revendication 17,
**caractérisée en ce que**
le bord formé (64c, 64d, 64g, 64h) est réalisé sous forme de perçage.

19. Prothèse articulaire selon une des revendications 1 à 18,
**caractérisée en ce que**
l'élément de guidage (13) comporte une section transversale essentiellement rectangulaire.

20. Prothèse articulaire selon une des revendications 1 à 19,
**caractérisée en ce que**
l'élément de guidage (13) comporte une section transversale avec des bords arrondis (71a-71d).

21. Prothèse articulaire selon la revendication 20,
**caractérisée en ce que**
l'élément de guidage (13) comporte une section transversale avec au moins une extrémité arrondie (72, 73).

22. Prothèse articulaire selon une des revendications 1 à 6,
**caractérisée en ce que**
l'élément de guidage (13) est relié à une tête de verrouillage (91) qui entre en prise dans le corps d'appui (30), afin de verrouiller le premier composant (1) dans le corps d'appui (30).

23. Prothèse articulaire selon la revendication 22,
**caractérisée en ce que**
l'axe longitudinal (92) de la tête de verrouillage (91) a pivoté d'un angle de pivotement (α) par rapport à l'axe longitudinal (17) de l'élément de guidage (13).

24. Prothèse articulaire selon la revendication 22 ou 23,
**caractérisée en ce que**
le corps d'appui (30) comporte au moins une surface de guidage (119, 120) contre laquelle la tête de verrouillage (91) est guidée lors du mouvement de translation du deuxième composant (2) par rapport au premier composant (1).

25. Prothèse articulaire selon une des revendications 22 à 24,
**caractérisée en ce que**
le deuxième composant comporte au moins un corps longitudinal (136, 137) qui comprend au moins une surface longitudinale (138, 139), et
**en ce que** la rotation de l'élément de guidage (13) par rapport au deuxième composant (2) est limitée par la butée d'au moins un côté longitudinal (106, 107) du corps d'appui (30) sur la surface longitudinale (138, 139).

26. Prothèse articulaire selon la revendication 25,
**caractérisée en ce que**
le côté longitudinal (106, 107) est réalisé sur une partie centrale (100) du corps d'appui (30).

27. Prothèse articulaire selon une des revendications 22 à 26,
**caractérisée en ce que**
le deuxième composant (2) comporte une surface courbée à plusieurs endroits (142) qui, lors d'un mouvement de translation du deuxième composant (2) par rapport au premier composant (1) et/ou d'une rotation de l'élément de guidage (13) par rapport au deuxième composant (2), limite le mouvement de la tête de verrouillage (91) pour limiter le mouvement de translation ou le mouvement de rotation.

28. Prothèse articulaire selon une des revendications 1 à 6,
**caractérisée en ce que**
l'élément de guidage (13) est relié à une tête de verrouillage (91) qui entre en prise dans le deuxième composant (2) pour relier le premier composant (1) au deuxième composant (2).

29. Prothèse articulaire selon la revendication 28,
**caractérisée en ce que**
la tête de verrouillage (91) entre en prise dans un espace borgne (166) du deuxième composant (2), un éloignement de la tête de verrouillage (91) étant empêché par arrêt de la tête de verrouillage (91) contre une surface de butée (170) de l'espace borgne (166).

30. Prothèse articulaire selon la revendication 28 ou 29,
**caractérisée en ce que**
le deuxième composant (2) comporte un trou oblong (160),
**en ce que** le corps d'appui (30) est relié à une broche (153) qui entre en prise dans le trou oblong (160), et
**en ce que** la rotation de l'élément de guidage (13) par rapport au deuxième composant (2) est limitée par la butée de la surface de l'enveloppe cylindrique (154) de la broche (153) contre les surfaces de butée (164, 165) du trou oblong (160).

31. Prothèse articulaire selon la revendication 30,
**caractérisée en ce que**
le trou oblong (160) est courbé de manière radiale par rapport à l'axe médian (161) du deuxième composant (2), l'axe médian (161) du deuxième composant (2) coïncidant à l'état monté de la prothèse articulaire au moins pour l'essentiel avec l'axe de rotation (150) du corps d'appui (30).

32. Prothèse articulaire selon une des revendications 22 à 31,
**caractérisé en ce que**
la surface (143) de la tête de verrouillage (91) est réalisée courbée, la courbure de la surface (143) étant adaptée à la courbure d'une surface courbée à plusieurs endroits (142) du deuxième composant (2), qui entoure au moins pour l'essentiel la tête de verrouillage (91).

33. Prothèse articulaire selon une des revendications 22 à 32,
**caractérisée en ce que**
la tête de verrouillage (91) comporte au moins une surface latérale (97, 98) qui, dans une position de rotation prescrite de la tête de rotation (91), est alignée sur une surface intérieure (109, 110) du corps d'appui (30) pour relâcher la prise de la tête de verrouillage (91) dans le corps d'appui (30).
